# EUROPEAN PATENT SPECIFICATION

(11) **EP 3 164 085 B1**
(45) Date of publication and mention of the grant of the patent: **10.04.2024**
(21) Application number: 15739100.4
(22) Date of filing: 26.06.2015
(51) Int. Cl.: A61B 17/12

(54) **COATED MEDICAL DEVICES**
BESCHICHTETE MEDIZINISCHE VORRICHTUNGEN
DISPOSITIFS MÉDICAUX ENROBÉS

(30) Priority: 27.06.2014 US 201414317587
(43) Date of publication of application: 10.05.2017
(73) Proprietor: Covidien LP, Mansfield, MA 02048 (US)
(72) Inventor: WAINWRIGHT, John, Foothill Ranch, California 92610 (US); MENDELSON, Todd, Mission Viejo, California 92691 (US); HAGGSTROM, Kurt, Mission Viejo, California 92692 (US); MOLAEI, Masoud, Laguna Niguel, California 92677 (US); SCHUMAN, Victoria, Minneapolis, Minnesota 55410 (US); SHEU, Min-Shyan, Chelmsford, Massachusetts 01824 (US)
(74) Representative: Gray, James
(86) International application number: PCT/US2015/037886
(87) International publication number: WO 2017/044067

(56) References cited:
- WO-A1-2014/152608
- US-A1- 2005 137 680
- US-A1- 2011 152 998
- US-A1- 2012 271 396

## Description

### BACKGROUND

Walls of the vasculature, particularly arterial walls, may develop areas of pathological dilatation called aneurysms. As is well known, aneurysms have thin, weak walls that are prone to rupturing. Aneurysms can be the result of the vessel wall being weakened by disease, injury, or a congenital abnormality. Aneurysms could be found in different parts of the body, and the most common are abdominal aortic aneurysms and brain or cerebral aneurysms in the neurovasculature. When the weakened wall of an aneurysm ruptures, it can result in death, especially if it is a cerebral aneurysm that ruptures.

Aneurysms are generally treated by excluding the weakened part of the vessel from the arterial circulation. For treating a cerebral aneurysm, such exclusion is done in many ways including: (i) surgical clipping, where a metal clip is secured around the base of the aneurysm; (ii) packing the aneurysm with small, flexible wire coils (micro-coils); (iii) using embolic materials to "fill" an aneurysm; (iv) using detachable balloons or coils to occlude the parent vessel that supplies the aneurysm; and (v) intravascular stenting.

Intravascular stents are well known in the medical arts for the treatment of vascular stenoses or aneurysms. Stents are prostheses that expand radially or otherwise within a vessel or lumen to provide therapy or support against blockage of the vessel. Methods for delivering these intravascular stents are also well known.

Various other implantable devices are used in surgical procedures. Often, these stents and other devices are coated with a coating material in order to achieve a desired therapeutic or other effect. Document WO 2014/152608 A1 is prior art according to Article 54 (3) EPC. Document US 2012/271396 A1 shows a stent with polymer coating.

### SUMMARY

The present invention is defined in the appended claims.

In accordance with some embodiments disclosed herein, a heat-treated device (e.g., stent) is provided that comprises an even coating that is substantially free of imperfections, such as lumps, fibers, webs, and/or other obstructions in the pores of the device. Such a device can be braided and/or have a flow diverting section.

Aspects of some embodiments disclosed herein recognize the existence of significant challenges in manufacturing a flow diverting device, such as a stent, that has a coating evenly applied over its surface. Hitherto, no process or device known to the Applicants has been developed that provides a device, such as a braided stent, with a coating that is evenly distributed over its surface, e.g., a coating that is devoid of imperfections, such as lumps, webs, fibers, or other obstructions in the pores of the stent.

Some embodiments disclosed herein provide a device with at least one flow diverting section that is coated and substantially free of webbing or that has a substantially uniform coating. Some embodiments relate to coating processes by which a device (e.g., a braided stent) can receive an even, generally imperfection-free coating. Additionally, some embodiments relate to methods of treatment using such coated devices (e.g., braided stents). Furthermore, some embodiments relate to one or more of the various advantageous features of the coated devices (e.g., braided stents).

For example, in some instances a medical device is described for treating an aneurysm. The device can comprise a tubular body comprising a plurality of braided filaments and configured to be implanted in a blood vessel. The body can be expandable to an expanded state for treatment of the aneurysm. The body can have a first section for spanning the neck of the aneurysm and a plurality of pores located between the filaments. The pores in the first section can have a first average pore size of less than about 500 microns when the body is in the expanded state. The first section can have a substantially complete coating, comprising a coating material, over the filaments. Further, the first section can be substantially free of webs formed between the braided filaments by the coating material.

The first section can comprise a length less than an entire length of the tube. The coating material on the first section can be generally uniform over the device or filaments. The coating can comprise an antithrombogenic material.

The medical device can further comprise a second section having a plurality of pores having a second average pore size greater than the first average pore size.

Further, some examples can describe a delivery system for treating an aneurysm. The system can comprise a microcatheter configured to be implanted into a blood vessel, a core assembly, extending within the microcatheter, having a distal segment, and the device extending along the core assembly distal segment.

In the invention, the medical device comprises a tubular member having a sidewall and a plurality of pores in the sidewall that are sized to inhibit flow of blood through the sidewall into an aneurysm to a degree sufficient to lead to thrombosis and healing of the aneurysm when the tubular member is positioned in a blood vessel and adjacent to the aneurysm. The device has an anti-thrombogenic coating distributed over the tubular member such that the pores are substantially free of webs formed by the coating.

The pores can have an average pore size that is the average size of the pores in the first section without the coating material. The coating can be substantially complete over the tubular member. The coating can be generally uniform over the device or tubular member. The tubular member comprises a plurality of braided filaments. The tubular member is substantially free of webs formed between the braided filaments by the coating. The flow diverting pores can extend over less than a longitudinal length that is less than a longitudinal length of the device or tubular member.

The device comprises a tubular member comprising a plurality of filaments that are braided together to form pores therebetween. The tubular member can have a flow diverting section configured to span the neck of the aneurysm. The device can also have a coating distributed over the flow diverting section. The coating is distributed completely over the flow diverting section substantially free of imperfections such that coated first and second longitudinal segments of the flow diverting section of approximately the same longitudinal lengths have approximately equal weights.

An exemplary medical device for treating an aneurysm can also be described that comprises a tubular member comprising a plurality of filaments, formed from a first material, that are braided together to form pores therebetween. The device can also comprise a coating material distributed over the filaments to form a coated flow diverting section that is substantially free of webs formed between the filaments by the coating material. The coating material can be distributed such that the device is significantly less thrombogenic than an uncoated device formed from the first material.

According to the invention is a anti-thrombogenic polymer. The coating material can be one or more of a variety of anti-thrombogenic materials or platelet aggregation inhibitors, or anti-thrombogenic polymers or monomers. Suitable coating materials include 2-Methacryloyloxyethyl phosphorylcholine (MPC, available as LIPIDURE^{™} from NOF Corporation of Tokyo, Japan). A suitable form of MPC is LIPIDURE^{™}-CM2056, or 2-Methacryloyloxyethyl phosphorylcholine-poly(n-butyl methacrylate). Additional suitable coating materials include PARYLENE C^{™}, or PARYLENE HT^{™}, both available from Specialty Coating Systems of Indianapolis, Indiana; BAYMEDIX^{™} available from Bayer AG of Leverkusen, Germany; BIOCOAT^{™} hyaluronic acid available from BioCoat, Inc. of Horsham, Pennsylvania; or polyethylene oxide. Other coating materials include heparin, heparin-like materials or derivatives, hirudin, H-Heparin, HSI-Heparin, albumin, phospholipids, streptokinase, tissue plasminogen activator (TPA), urokinase, hyaluronic acid, chitosan, methyl cellulose, poly(ethylene oxide), poly(vinyl pyrrolidone), endothelial cell growth factor, epithelial growth factor, osteoblast growth factor, fibroblast growth factor, platelet derived growth factor or angiogenic growth factor.

In some embodiments, the pores can have an average pore size that is less than or equal to about 500 microns. The pores can have an average pore size that is less than or equal to about 320 microns. The pores can have an average pore size that is from about 50 microns to about 320 microns. The pores can have a pore size that is generally constant. The pores can have an average pore size that is measured using an inscribed circle diameter.

Some exemplary processes disclosed herein comprise mounting or maintaining a braided, flow diverting device (e.g., stent) in a longitudinally stretched configuration during the coating process in order to prevent coating imperfections, such as webbing. The longitudinally stretched configuration can enable individual filaments of the braided device to overlap each other at angles of between about 75 degrees and about 105 degrees with respect to each other. The longitudinally stretched configuration can enable individual filaments of the braided device to overlap each other at angles of between about 80 degrees and about 100 degrees with respect to each other. Further, the longitudinally stretched configuration can enable individual filaments of the braided device to overlap each other at angles of between about 85 degrees and about 95 degrees with respect to each other. Furthermore, the longitudinally stretched configuration can enable individual filaments of the braided device to overlap each other approximately perpendicularly or at a generally right angles with respect to each other. In some examples, therefore, the longitudinally stretched configuration can orient the individual filaments to create a pattern of quadrilaterals, such as squares, rectangles, parallelograms, rhombuses, trapezoids, etc.

Further, some examples of processes disclosed herein comprise dipping a longitudinally stretched braided device (e.g., stent) in a coating solution and thereafter air knifing the dipped device. The process of air knifing can comprise applying at least one powerful jet of air to remove or blow off any excess solution from the device. The air jet(s) of the air knife(s) can be applied in a direction that is generally transverse, such as orthogonal, relative to the longitudinal axis of the device. The air knife can be stationary while the device is moved or it can move along and/or about the device as the device remains stationary.

In some examples, the longitudinally stretched braided device (e.g., stent) can alternatively be coated with a coating solution using a spraying operation. In some examples, while the device is being coated, the device can be rotated about its central longitudinal axis to ensure even application of the air jet or sprayed coating.

For example, a method of coating a stent is described wherein the method comprises: attaching first and second ends of the stent with upper and lower connectors of a holder device, the stent comprising a flow diverting section; dipping the stent into a coating material to coat a first section of the stent; and removing excess coating material from the stent such that the stent is free of webs formed by the coating material.

The method can be performed such that the flow diverting section comprises a plurality of pores having an average pore size that is less than or equal to about 500 microns. Further, the average pore size can be less than or equal to about 320 microns. The average pore size can be from about 50 microns to about 320 microns.

The method can be performed such that dipping the stent into the coating material comprises dipping less than an entire length of the stent into the coating material to maintain an open air pocket adjacent to the stent first end. Further, attaching the first and second ends of the stent can comprise attaching the stent to the holder device such that the stent is held between the upper and lower connectors in a radially collapsed, longitudinally elongated state. The stent can comprise a plurality of braided filaments, and the stent filaments can cross each other at substantially right angles when the stent is held in the elongated state. Further, the elongated state can be achieved when the stent filaments cross each other at angles in an angular range from about 80° to about 110°. In addition, the stent can comprise a plurality of braided filaments, and the coating material on the stent can be substantially free of webs such that the coating material does not bridge between adjacent filaments.

Additionally, some examples of the coating (e.g., dipping, spraying, etc.) processes disclosed herein can be performed using a cantilevered fixture. A cantilevered fixture, which can be suitable for use in, for example, a dipping process, can also be designed to be suitable for use in air knifing processes, when necessary. In order to be suitable for air knifing processes, however, the cantilevered fixture can beneficially be configured to resist deflection, such as the "pendulum effect" that occurs when an air jet is applied to the mounted device, and the device and the fixture begin to move back and forth harmonically, off-axis. Further, the cantilevered fixture can also beneficially be configured to mount the device thereto without passing through a lumen of the device or otherwise interfering with air flow from the air knife. Furthermore, the cantilevered fixture can also beneficially avoid contact with the device in order to prevent wicking or removing solution from the surface of the device.

Accordingly, in some examples, the cantilevered fixture can be both rigid and lightweight. For example, a lower (free) end of the cantilevered fixture can be lighter weight than an upper (cantilevered) end of the cantilevered fixture.

Further, in some examples, the cantilevered fixture can comprise first and second ends that engage with corresponding ends of the device (e.g., stent) and a fixture body that extends between the first and second ends and outside of a device lumen when the device is mounted to the fixture. The first end can comprise one or more clips or protrusions that engage a first end of the device, such as by pinching, grasping, friction, and/or hook and loop, or other mechanical fastening means. The second end can comprise one or more clips or protrusions that engage an opposing end of the device, such as by pinching, grasping, friction, and/or a hook and loop or other mechanical fastening means. The first and second ends can be upper or lower ends of the cantilevered fixture. The first and second ends can be spaced apart sufficiently to maintain the device in a longitudinally stretched configuration when mounted on the cantilevered fixture.

In accordance with some examples, a method of coating a stent is described in which the method comprises: attaching a first end of the stent with an upper connector of a holder device, the stent comprising a plurality of braided filaments; attaching a second end of the stent with a lower connector of the holder device such that the stent is held between the upper and lower connectors in a radially collapsed, longitudinally elongated state, the stent filaments crossing each other at substantially right angles when the stent is held in the elongated state; while maintaining the stent in the elongated state, dipping the stent into a coating material to coat a first section of the stent; and removing excess coating material from the stent.

The elongated state can be achieved when the stent filaments cross each other at angles ranging from about 80° to about 110°. Further, the elongated state can be achieved when the stent filaments cross each other at angles in a range from about 85° to about 95°.

The method can be performed such that removing excess coating material comprises applying a stream of gas to the stent filaments. The stream of gas can be of a sufficient strength to remove excess coating material from the stent. Further, removing excess coating material can comprise rotating the stent and holder device while applying a stream of gas to impinge upon an outer surface of the stent. Furthermore, the method can comprise drying the coating material applied to the stent. For example, the drying can comprise drying the stent in an oven at between from about 50° to about 80° for between from about 5 minutes to about 1 hour, and in some embodiments, at about 60° for about 15 minutes.

The method can be performed such that dipping the stent into the coating material comprises dipping less than an entire length of the stent into the coating material to maintain an open air pocket adjacent to the stent first end. Further, the holder device upper and lower connectors can be interconnected such that the holder device is in a cantilevered configuration during the dipping and removing steps.

In some examples, a method of coating a stent can be described that comprises: attaching a first end of the stent with an upper connector of a holder device, the stent comprising a plurality of braided filaments and a plurality of pores located between the filaments; attaching a second end of the stent with a lower connector of the holder device such that the stent is held between the upper and lower connectors in a radially collapsed, longitudinally elongated state in which the filaments are oriented to substantially maximize an average inscribed area of the pores; while maintaining the stent in the elongated state, dipping the stent into a coating material to coat a first section of the stent; and removing excess coating material from the stent.

The method can be performed such that the maximum average inscribed area of the pores is achieved when the filaments cross each other at substantially right angles. The maximum average inscribed area of the pores can be achieved when the pores are substantially square. The maximum average inscribed area of the pores can be achieved when the filaments cross each other at angles in a range from about 80° to about 110°. Further, the maximum average inscribed area of the pores can be achieved when the filaments cross each other at angles in a range from about 85° to about 95°.

Additional features and advantages of the subject technology will be set forth in the description below, and in part will be apparent from the description, or may be learned by practice of the subject technology. The advantages of the subject technology will be realized and attained by the structure particularly pointed out in the written description and embodiments hereof as well as the appended drawings.

It is to be understood that both the foregoing general description and the following detailed description are exemplary and explanatory and are intended to provide further explanation of the subject technology.

### BRIEF DESCRIPTION OF THE DRAWINGS

The accompanying drawings, which are included to provide further understanding of the subject technology and are incorporated in and constitute a part of this specification, illustrate aspects of the disclosure and together with the description serve to explain the principles of the subject technology.
Figure 1 is a perspective view of a device, illustrated as a stent, having a coating applied using a prior art method, illustrating webbing of the coating.
Figure 2 is an enlarged view of a stent having a coating applied using a prior art method, illustrating delamination of the coating.
Figures 3A-3C are enlarged views of a stent having a coating applied using a prior art method, illustrating accumulation of excess coating material and webbing.
Figure 4 is a side view of a stent comprising a coating, according to some embodiments.
Figure 5A is an enlarged view of the stent shown in Figure 4, according to some embodiments.
Figures 5B-5C are detail views of a pore of the stent of Figure 4, in various conditions.
Figure 6 is a side view of a coating system having a stent mounted thereon in a first position in preparation for coating the stent, according to some embodiments.
Figure 7A is a side view of the coating system of Figure 6, wherein the stent has been immersed in a coating solution, according to some embodiments.
Figure 7B is a detail view of the dipping procedure near an upper end of the stent being dipped.
Figure 8A is a side view of the coating apparatus of Figure 6, wherein the stent is removed from the coating solution and coating solution is removed, according to some embodiments.
Figure 8B is a detail view of an air knifing procedure being carried out on the stent with the coating system of Figure 6.
Figure 9A is a side view of the coating apparatus of Figure 6, wherein the stent has been returned to the first position.
Figure 9B is a detailed side view of a stent mounting apparatus for use with the coating system of Figure 6.
Figure 9C is a detailed front view of the stent mounting apparatus of Figure 9B.
Figure 9D is a detailed view of a lower fixture of the stent mounting apparatus of Figures 9B-9C.
Figure 10 is a side view of the mounting apparatus of Figures 9B-9D, with a stent mounted thereon.
Figure 11 is an enlarged view of an upper bracket of the mounting apparatus of Figures 9B-10, according to some embodiments.
Figure 12 is an enlarged view of a lower bracket of the mounting apparatus of Figures 9B-11, according to some embodiments.
Figure 13 is an enlarged view of a stent mounted on the mounting apparatus of Figures 9B-12 in a stretched configuration, according to some embodiments.
Figure 14A is an enlarged view of a coated stent in a relaxed configuration, demonstrating no visible webbing or delamination, according to some embodiments.
Figure 14B is a detailed view of a filament crossing point of a coated stent.
Figure 15 is a chart of weight gain measured in series of longitudinal sections of six coated stents.
Figures 16A-16C are views of a stent coated according to some embodiments, demonstrating no webbing or delamination of the coating.
Figures 17A-17C are views of another stent coated according to some embodiments, demonstrating no webbing or delamination of the coating.
Figures 18A-18C are views of yet another stent coated according to some embodiments, demonstrating no webbing or delamination of the coating.
Figures 19A-19C are views of yet another stent coated according to some embodiments, demonstrating no webbing or delamination of the coating.

### DETAILED DESCRIPTION

In the following detailed description, numerous specific details are set forth to provide a full understanding of the subject technology. It should be understood that the subject technology may be practiced without some of these specific details. In other instances, well-known structures and techniques have not been shown in detail so as not to obscure the subject technology. Further, although the present disclosure may refer to embodiments in which the apparatus is a stent, aspects of the embodiments disclosed herein can be used with any implantable device, such as coils, filters, scaffolds, self-expanding and balloon-expandable stents, and other devices.

In accordance with some embodiments disclosed herein, a heat-treated device (e.g., stent) is provided that comprises an even coating that is substantially free of imperfections, such as lumps, fibers, webs, and/or other obstructions in the pores of the device. Further, in some embodiments, such a device can be braided and/or have a flow diverting section.

To the Applicants' knowledge, the devices and methods disclosed herein have not been available or possible based on prior devices and methods of manufacture. In general, prior coated devices, such as stents, have demonstrated various coating imperfections and resultant disadvantages.

Among these disadvantages, "webbing," delamination, and uneven layering of coating material pose significant risks. "Webbing" of coating material occurs when the coating material spans or extends between filaments of a device to create a thin, localized film of coating material between the filaments. Delamination occurs when coating material peels away from or is not bound to the device filaments. Uneven layering of coating material can exacerbate the effects of delamination and webbing, creating large pieces of coating that do not easily dissolve or pass through a blood vessel when such pieces are dislodged. Webbing and delamination create a significant risk to proper blood flow if that coating material is dislodged or breaks free from the device filaments. If this occurs, the dislodged coating material can create or contribute to blockages in the blood vessel. This is a dangerous condition that can result from certain prior art devices and coating processes.

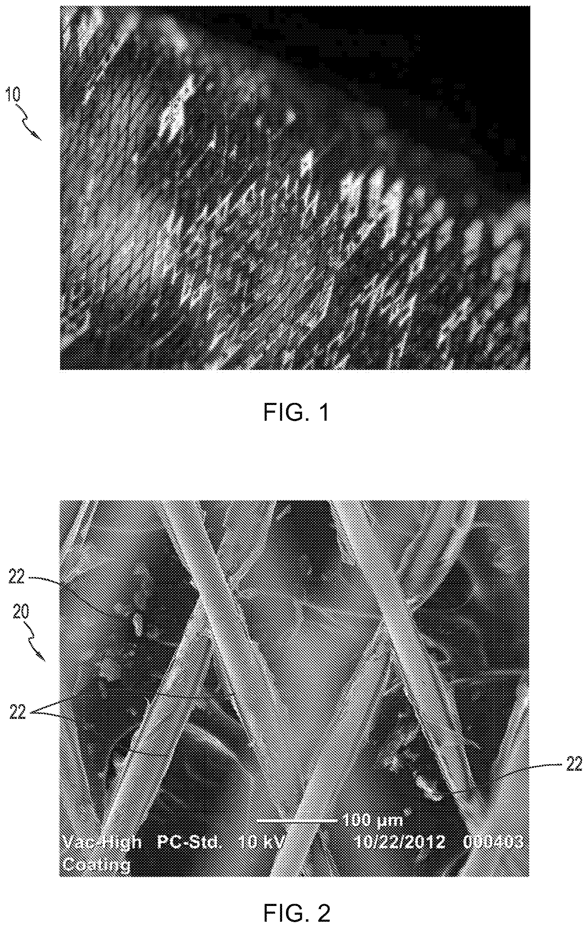

For example, referring to Figure 1, a braided stent 10 illustrates problems that can arise when the stent 10 is coated with a coating material. As shown, the coating material (which appears in Figure 1 as the relatively brighter portions of the stent 10) is distributed generally unevenly along the braided stent 10 and has spanned between adjacent filaments to produce "webs" or "webbing" between the filaments of the braided stent 10.

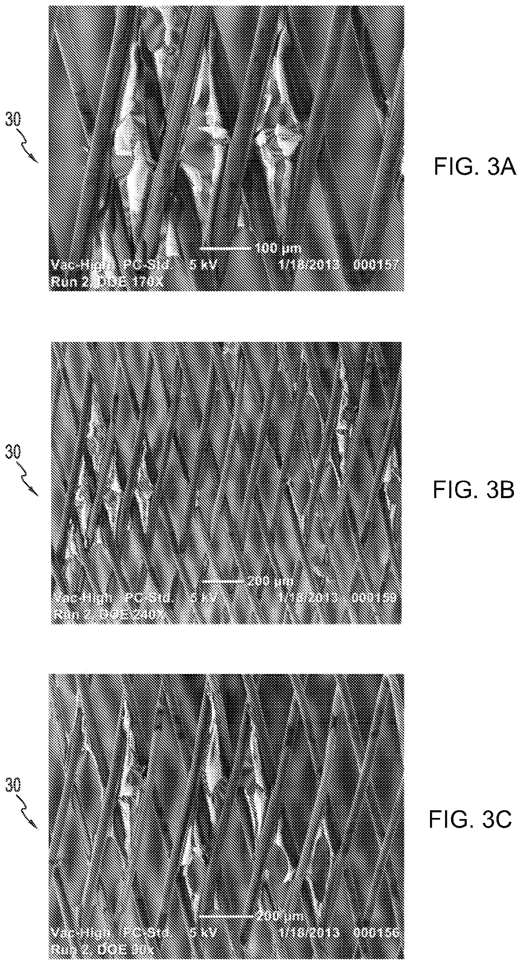

Figure 2 is a high magnification view of a braided stent 20 coated with a coating material 22 that has delaminated. The delaminated coating material 22 can easily break free from the stent, as discussed below. Figures 3A-C also illustrate other views of a stent 30 having excess coating and delamination.

Typically, a braided vascular device such as a stent is braided from filaments which are formed from metal alloys and/or other high-temperature materials. The resulting braid is then heat-treated or "heat-set" at high temperature in order to reduce internal stresses in the filaments and increase the self-expanding capability of the stent. The prevalence of heat treatment and need for self-expanding properties in the manufacture of braided vascular devices and stents negates the possibility of "pre-coating" the individual filaments with a low-temperature material such as a polymer, a drug or a drug carrier and then braiding the pre-coated filaments to form a device which must then be heat-treated. Truly, a person of skill has had no expectation of successfully braiding filaments that have been pre-coated with any of the low-temperature materials that make up the bulk of useful coatings, and later heat setting the braided device to create a coated, heat-set device because of the significant damage the heat would cause to the coating.

Furthermore, no prior device or method of manufacturing known to the Applicants has been able to produce a heat-treated, coated, braided implantable device with a small pore size that is free of the disadvantages of webbing, delamination, and uneven layering of coating material, which is now possible in accordance with some implementations of the present disclosure. Again, given the exceedingly small pore size, a person of skill has had no expectation of successfully coating such a device evenly and without the disadvantages of webbing, delamination, or other coating imperfections. Figures 1-3C demonstrate the difficulty of coating such small-pore-size devices.

Indeed, to the present knowledge of the Applicants, no prior devices or methods of manufacture have been developed that produce a coated device (e.g., stent) having a flow diverting section that is substantially free of the disadvantages noted above. As discussed herein, a flow diverting section can have pores with a "flow diverting pore size." A "flow diverting pore size" can refer to an average pore size of pores (in at least a section of a device) that is sufficiently small enough to interfere with or inhibit fluid exchange through the pores of that section. For example, a device (e.g., stent) can have an active section or a flow diverting section with a flow diverting pore size when the pores of the section are sized to inhibit flow of blood through the sidewall into an aneurysm to a degree sufficient to lead to thrombosis and healing of the aneurysm when the tubular member is positioned in a blood vessel and adjacent to or across the neck of the aneurysm.

For example, a flow diverting pore size can be achieved when pores in the flow diverting or active section (or in the stent as a whole) have an average pore size of less than about 500 microns when the device (e.g., stent) is in the expanded state. According to the invention, the pore size is less than or equal to about 500 microns. (When "expanded state" is used herein to specify braided stent parameters such as pore sizes, the expanded state is one that the stent will self-expand to without any external expansive forces applied, and without any external longitudinal stretching or compressive forces applied. For simplicity of measurement, this expanded state can be one that the stent will self-expand to within a straight glass cylindrical tube with an inside diameter that is smaller than the maximum diameter to which the stent will self-expand in the absence of any containment or external forces.) In some embodiments, the average pore size can be less than about 320 microns. Indeed, because of such exceedingly small average pore sizes, any known prior device or attempt at manufacturing such a device resulted in substantial webbing, delamination, and uneven application of coating to the device. In contrast, some embodiments disclosed herein enable and provide a device and methods of manufacturing in which the device has a flow diverting section that is substantially free of webbing, delamination, and other coating deficiencies.

Accordingly, some embodiments provide a device, such as a braided stent, that can have a flow diverting section or other portion of the devicethat provides embolic properties so as to interfere with blood flow in (or into) the body space (e.g., an aneurysm) in (or across) which the device is deployed. The porosity and/or pore size of one or more sections of the device can be selected to interfere with blood flow to a degree sufficient to thrombose the aneurysm or other body space.

For example, some embodiments provide a device (e.g., stent) that can be configured to interfere with blood flow to generally reduce the exchange of blood between the parent vessel and an aneurysm, which can induce thrombosis of the aneurysm. A device (or a device component, such as a sidewall of a stent or a section of such a sidewall) that thus interferes with blood flow can be said to have a "flow diverting" property.

Additionally, in some embodiments, a device (e.g., stent) can be provided with a porosity in the range of 5%-95% may be employed in the expanded braid. In some embodiments, a porosity in the range of 30%-90% may be employed. Further, a porosity in the range of 50%-85% may be employed.

Further, in some embodiments, a device (e.g., stent) can be provided with a pore size in the range of 20-300 microns (inscribed diameter). In some embodiments, a pore size in the range of 25-250 microns (inscribed diameter) may be employed. In some embodiments, a pore size in the range of 50-200 microns (inscribed diameter) may be employed.

Methods of treatment and methods of manufacturing embodiments of the devices (e.g., stents) disclosed herein are also provided. Therefore, various embodiments of the devices disclosed herein address the problems and complications associated with unevenly and improperly coated devices (e.g., stents) and provide novel processes for manufacturing and using such devices.

Some embodiments of processes disclosed herein comprise mounting or maintaining a braided device (e.g., stent) in a longitudinally stretched configuration during the coating process. Such a device can have an expanded configuration in which the pores thereof are generally circumferentially elongated, which results in a decreased pore size or a relatively "closed" configuration. In contrast, the pore size is increased or in a relatively "open" configuration when the device is in the longitudinally stretched configuration. In the longitudinally stretched configuration, many, if not all, of the pores of the device can be opened to an enlarged pore size, or to a generally maximum pore size.

For example, in some embodiments, the longitudinally stretched configuration can open the pores by orienting the individual filaments of the device to create a pattern of open-pore quadrilaterals, such as squares, rectangles, parallelograms, rhombuses, trapezoids, etc., which can allow the pore size to be generally maximized. Further, the quadrilaterals can be formed by filaments that cross at angles from about 0° to about 15° from a right angle. In some embodiments, the angles can be from about 0° to about 10° from a right angle. In some embodiments, the angles can be from about 0° to about 5° from a right angle. Additionally, in some embodiments, the filaments can form right-angled quadrilaterals, such as squares and rectangles, which allows the pore size to be maximized. However, not every pore shape circumscribed by the filaments may be a right-angled quadrilateral, and some variation between pores in the same or different sections of a device is possible.

Further, some embodiments of processes disclosed herein comprise dipping a longitudinally stretched braided device (e.g., stent) in a solvent and thereafter "air knifing" the dipped device to remove or blow off any excess solvent from the device. The air jet(s) of the air knife(s) can be applied in a direction that is generally transverse, such as orthogonal, relative to the longitudinal axis of the device. The air knife can be stationary while the device is moved, or it can move along or about the device as the device remains stationary.

Additionally, some embodiments also provide a coating fixture which can be used during the coating (e.g., dipping, spraying, etc.) processes to optimize the coating and drying process and prevent inadvertent wicking of the coating material away from the coated device (e.g. stent), which can result from undesired contact with the coated device (e.g., stent) during the process. Some embodiments of the coating fixture can be both rigid and/or lightweight. Further, in some embodiments, the coating fixture can comprise first and second ends that engage with corresponding ends of the device and a fixture body that extends between the first and second ends and outside of a device lumen when the device is mounted to the fixture. The first and second ends can be spaced apart sufficiently to maintain the device in a longitudinally stretched configuration when mounted on the cantilevered fixture.

The device (e.g., stent) can take the form of a vascular occluding device, a revascularization device and/or an embolization device. In some embodiments, the device can be an expandable stent made of two or more filaments. The filaments can be formed of known flexible materials including shape memory materials, such as nitinol, platinum and stainless steel. In some embodiments, the filaments can be round or ovoid wire. Further, the filaments can be configured such that the device is self-expanding. In some embodiments, the device can be fabricated from a first group of filaments made of platinum alloyed with 8% tungsten, and a second group of filaments made of 35N LT (cobalt nickel alloy, which is a low titanium version of MP35N alloy). In other embodiments, one or more of the filaments can be formed of a biocompatible metal material or a biocompatible polymer.

The wires or filaments can be braided into a resulting lattice-like structure. In at least one embodiment, during braiding or winding of the device (e.g., stent), the filaments can be braided using a 1-over-2-under-2 pattern. In other embodiments, however, other methods of braiding can be followed, without departing from the scope of the disclosure. The device can exhibit a porosity configured to reduce haemodynamic flow into and/or induce thrombosis within, for example, an aneurysm, but simultaneously allow perfusion to an adjacent branch vessel whose ostium is crossed by a portion of the device. As will be appreciated, the porosity of the device can be adjusted by "packing" the device during deployment, as known in the art. The ends of the device can be cut to length and therefore remain free for radial expansion and contraction. The device can exhibit a high degree of flexibility due to the materials used, the density (i.e., the porosity) of the filaments, and the fact that the ends of the wires or filaments are not secured to each other.

Information regarding additional embodiments, features, and other details of the devices, methods of use, and other components that can optionally be used or implemented in embodiments of the occlusion devices described herein, can be found in Applicants' co-pending applications U.S. Patent Application Nos. 12/751,997, filed on March 31, 2010; 12/426,560, filed on April 20, 2009; 11/136,395, filed May 25, 2005; 11/420,025, filed May 24, 2006; 11/420,027, filed May 24, 2006; 12/425,604, filed April 17, 2009; 12/896,707, filed October 1, 2010; 61/483,615, filed May 6, 2011; 61/615,183, filed March 23, 2012; 13/614,349, titled Methods and Apparatus for Luminal Stenting, filed on September 13, 2012 (reference HKN-02608 (1), 080373-0366); 13/692,021, titled Methods and Apparatus for Luminal Stenting, filed on December 3, 2012 (reference HKN-02608 (2), 080373-0377); and 13/664,547, titled Methods and Apparatus for Luminal Stenting, filed on October 31, 2012 (reference HKN-02608 (3), 080373-0498).

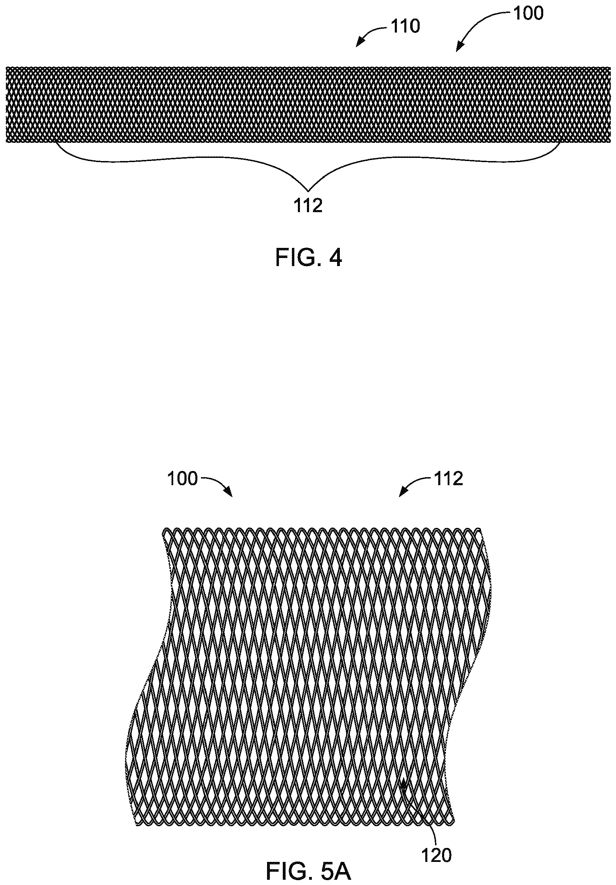

Figure 4 illustrates a tubular, self-expanding device, shown as a stent 100, comprising a coating 110 disposed along at least a portion thereof. The tubular stent 100 comprises an elongate hollow body which can be formed from a plurality of braided filaments. Some embodiments disclosed herein can comprise a coating along the entire length of the stent or merely along only a portion thereof. The stent 100 can comprise a flow diverting portion 112. The flow diverting portion 112 can comprise a plurality of pores that have a flow diverting pore size; instead of or in addition to this property, the flow diverting portion 112 can have a flow diverting porosity. The flow diverting portion 112 can comprise a portion of the stent 100, or the entire stent. The flow diverting pore size can be an average pore size within a relevant portion of the stent, e.g. within the flow diverting portion 112 or a portion thereof, or a "computed" pore size, one that is computed from measured or nominal basic stent parameters such as braid angle, number of filaments, filament size, filament diameter, stent diameter, longitudinal picks per inch, radial picks per inch, etc. Such a computed pore size can be considered to be one type of average pore size. The flow diverting pore size can be within a size range that that interferes with or inhibits blood flow through the sidewall of the stent 100, for example, between the parent vessel and an aneurysm sufficient to induce or lead to thrombosis of the aneurysm. The coating can be disposed partially or entirely along the flow diverting portion 112, or along another portion of the stent 100.

In some embodiments, the pores of the flow diverting portion 112 can have an average pore size of less than 500 microns (inscribed diameter), or in the range of 20-300 microns (inscribed diameter). Further, the average pore size can be in the range of 25-250 microns (inscribed diameter). Furthermore, the average pore size can be in the range of 50-200 microns (inscribed diameter). The stent 100 can be expandable from an unexpanded diameter to an expanded diameter; the unexpanded diameter can optionally be 5mm or less, or 4mm or less, or 3mm or less, or 2mm or less, or other unexpanded diameters, and/or the expanded diameter can optionally be 2mm or more, or 3mm or more, or 4mm or more, or 5mm or more, or other expanded diameters. The unexpanded diameter can be less than the expanded diameter.

The average pore size of the pores in the flow diverting portion 112 can be the average size of the pores measured with or without coating material disposed thereon. Thus, the average pore size of the flow diverting portion of a bare stent can be within the flow diverting ranges. Further, the average pore size of the flow diverting portion of a coated stent can be within the flow diverting ranges. Furthermore, the flow diverting portion 112 can comprise pores having sizes above or below the range of the average pore size.

Figure 5A illustrates an enlarged view of a section of the flow diverting portion 112 of the stent 100. In this embodiment, the flow diverting portion 112 comprises a plurality of filaments 120 that are braided together to form the tubular body of the stent 100. Figure 5A illustrates the self-expanding stent 100 in an expanded or relaxed state. In this expanded of relaxed state, the filaments 120 cross each other to form the pores of the stent 100.

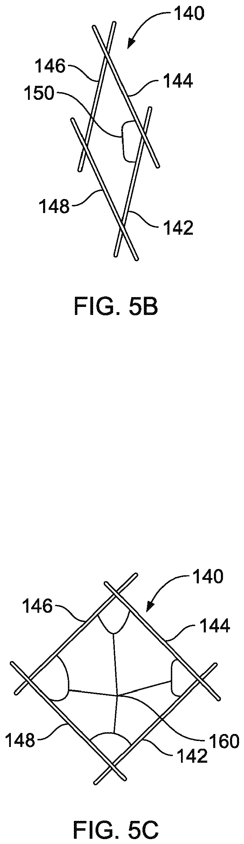

Figure 5B illustrates a single pore 140 of the flow diverting section 112 when in the relaxed state. The pore 140 is formed by a plurality of filaments 142, 144, 146, and 148. As shown, the filaments 142, 144 cross each other to form an obtuse angle 150. In some embodiments, the obtuse angle 150 can be from about 110° to about 170°. Further, the obtuse angle 150 can be from about 120° to about 165°. Further, the obtuse angle 150 can be from about 130° to about 160°, and in some embodiments, the obtuse angle 150 can be about 150°.

Accordingly, the size or configuration of the pore 140 is "closed" or relatively small in the expanded or relaxed state shown in Figure 5B when compared with the relatively "open" size of the pore 140 when the stent 100 is in a longitudinally stretched configuration, as shown in Figure 5C. Figure 5C illustrates that the filaments 142, 144, 146, and 148 each cross each other at angles 160 that approximate a right angle, e.g. within from about 0° to about 15° from a right angle. In some embodiments, the angles 160 can be from about 0° to about 10° from a right angle. In some embodiments, the angles 160 can be from about 0° to about 5° from a right angle.

Additionally, in order to maximize the pore size, in some embodiments, the filaments can form right-angled quadrilaterals, such as squares and/or rectangles. However, not every pore shape circumscribed by the filaments may be a right-angled quadrilateral, and some variation between pores in the same or different sections of a stent is possible.

A device can be prepared according to some embodiments by braiding a plurality of filaments to form a braided stent, filter, or other braided device. The device can then be cleaned and heat treated, if necessary, to impart desired characteristics to the device. Thereafter, the device can be coated using aspects of the methods disclosed herein.

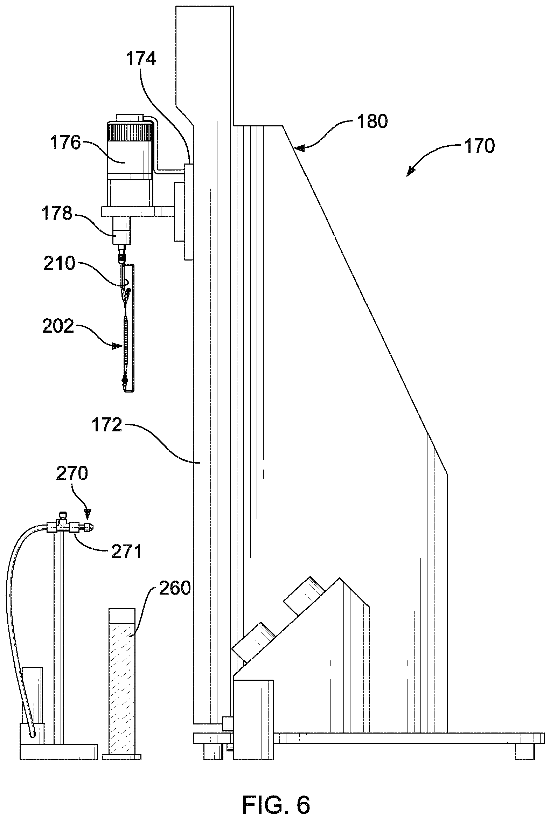

Figures 6-12 illustrate aspects of a coating apparatus and method that can be used in some embodiments to coat the stent 100, or any other embodiments of stents or other devices disclosed herein. Figure 6 illustrates a coating system 170 that generally comprises a handling system 180, a mounting apparatus 210 that is held by the handling system 180 and in turn holds the stent 202, an open-topped container (e.g., a beaker) of coating solution 260, and an air knife 270.

The handling system 180 is generally configured to rotate and move the stent 202 and mounting apparatus 210 vertically up and down. Thus the handling system 170 includes a powered linear actuator 172 that is oriented vertically and further includes a carriage 174 that can move up and down on, and under the power of, the linear actuator 172. On the carriage 174 is mounted an electric motor 176; when powered the motor 176 rotates a chuck 178. The chuck 178 detachably grips an upper end of the mounting apparatus 210 so that the motor 176 rotates the mounting apparatus 210 and stent 202 about a generally vertical axis when the motor 176 is energized.

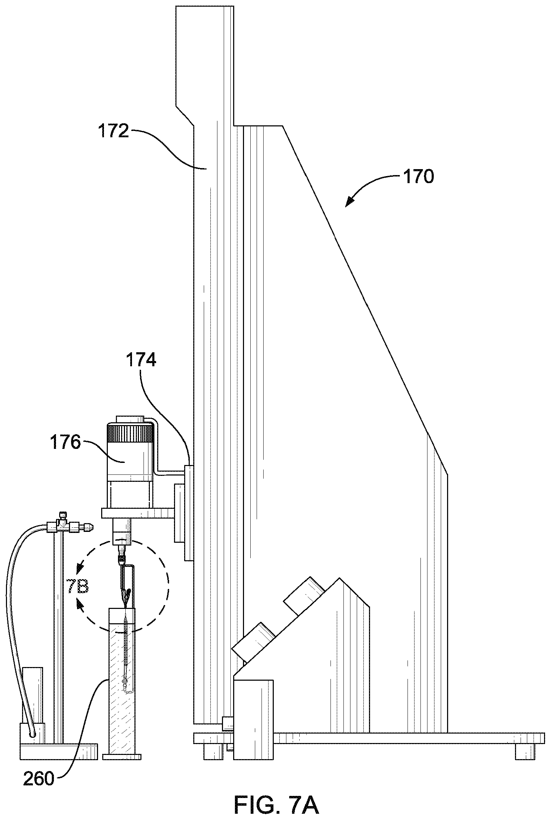

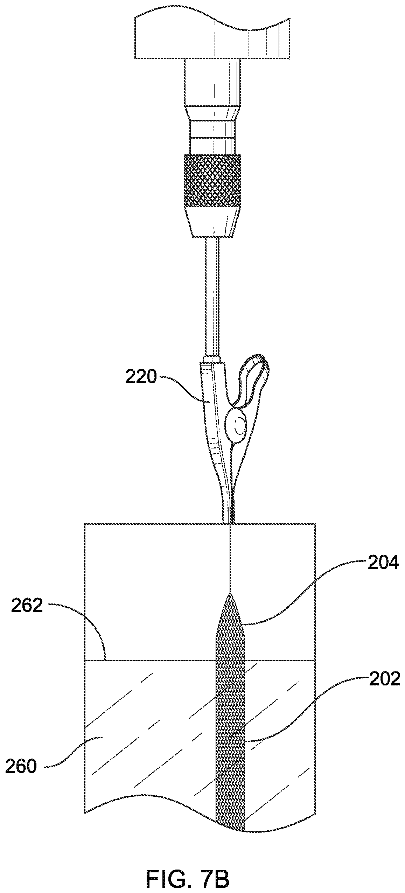

The coating solution 260 is positioned beneath the motor 176 and mounting apparatus 210, and aligned generally with the mounting apparatus 210 and the rotational axis of the motor 176. Therefore, a downward movement of the carriage 174 (and with it the motor 176 and mounting apparatus 210) along the linear actuator 172 will cause the lower portions of the mounting apparatus 210 and stent 202 to be immersed in the coating solution 260 (see Figures 7A, 7B).

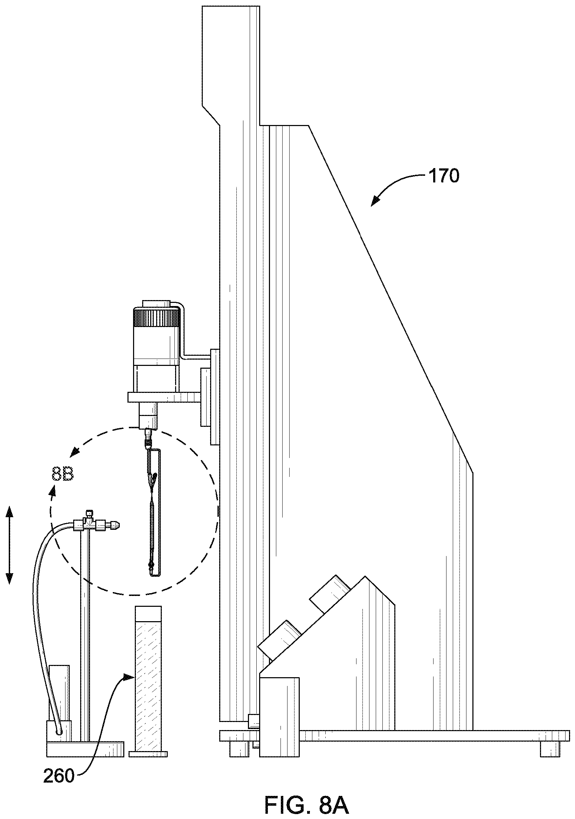

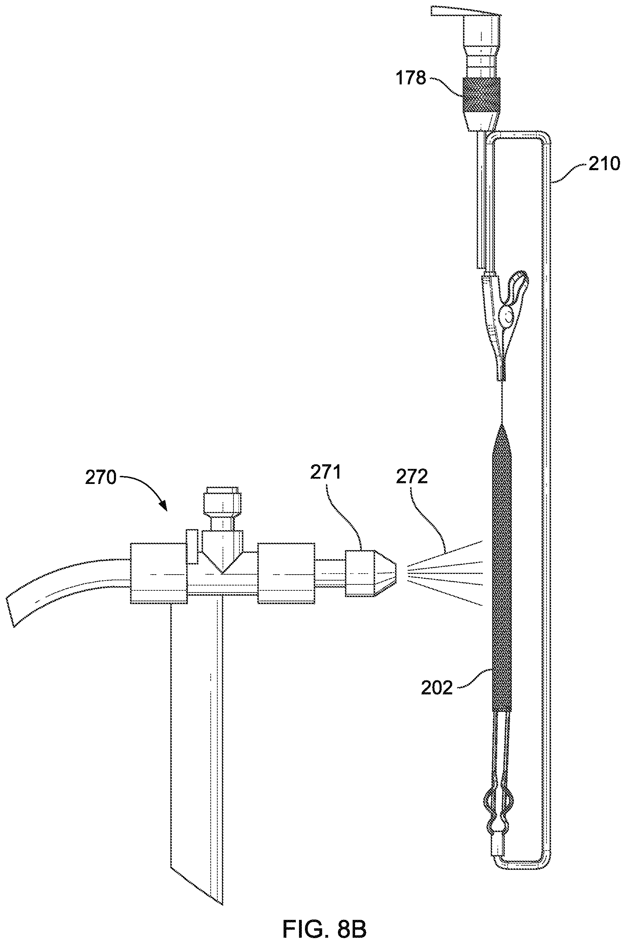

The air knife 270 comprises a nozzle 271 that is connected via a hose to a source of pressurized air or other pressurized gas (not shown). The nozzle 271 is positioned and oriented so that, upon activation of the air knife 270, the nozzle directs an air jet or air stream 272 (see Figure 8B) that strikes the stent 202 when the carriage 174 and motor 176 are positioned along or moving through a particular "air knifing" region of the linear actuator 172.

The coating system 170 can be used to coat a stent generally as follows. The process begins with the coating system in the start position shown in Figure 6. The linear actuator 172 is energized to lower the carriage 174, and with it the motor 176, mounting apparatus 210 and stent 202, until the lower portions of the apparatus 210 and stent 202 are submerged in the coating solution 260 (see Figures 7A, 7B). The mounting apparatus 210 and stent 202 are left in the solution 260 momentarily before the linear actuator 172 is energized again to raise the stent 202, now wet with coating solution, toward the air knife 270. When the stent 202 is fully out of the coating solution 260, the actuator 172 pauses for a brief pre-drying period before moving the stent 202 into the air stream 272. Once the pre-drying period is over, the stent is moved upward into the air stream 272. While the air stream 272 is striking the stent 202, the stent is simultaneously rotated and reciprocated vertically (via the motor 176 and actuator 172, respectively) relative to the nozzle 271 so that the stent is moved back and forth through the air stream. Thus, substantially all of the outer surface of the stent 202 is moved across the nozzle 271 and exposed to the air stream 272 during the knifing process, and any excess coating solution is blown off.

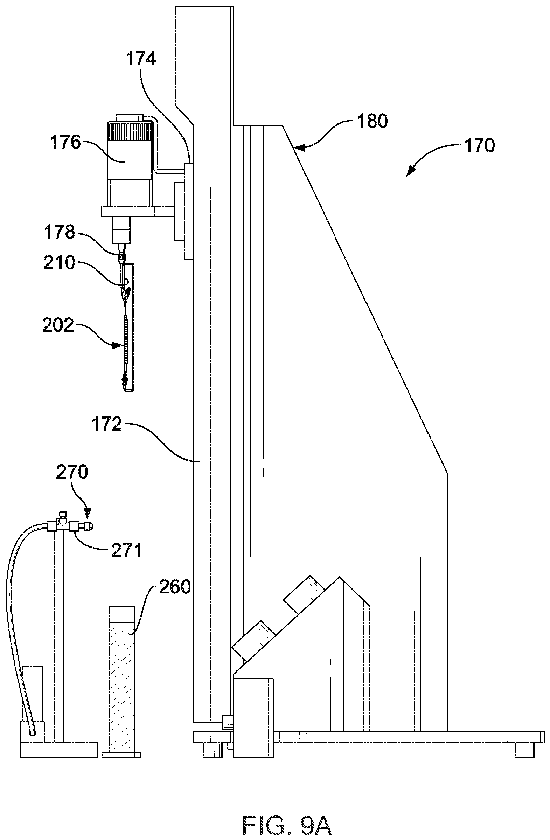

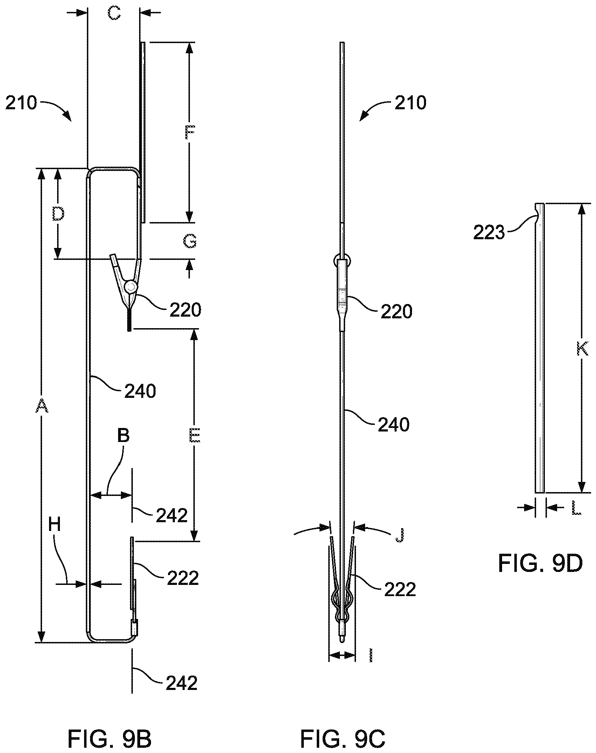

After air knifing is complete, the stent 202 is then raised out of the stream 272 via the actuator 172 (and/or the air knife 270 is deactivated), as shown for example in Figure 9A. The mounting apparatus 210 is now removed from the motor 176 by loosening the chuck 178. The stent 202 can be allowed to dry further while still mounted on the mounting apparatus, either at room temperature or at elevated temperature in a drying oven. Once the stent is dry, it can be removed from the mounting apparatus 210 and cut to its final length by trimming off one or both ends of the stent.

More specific aspects of the coating system 170 and methods of coating will now be discussed in greater detail.

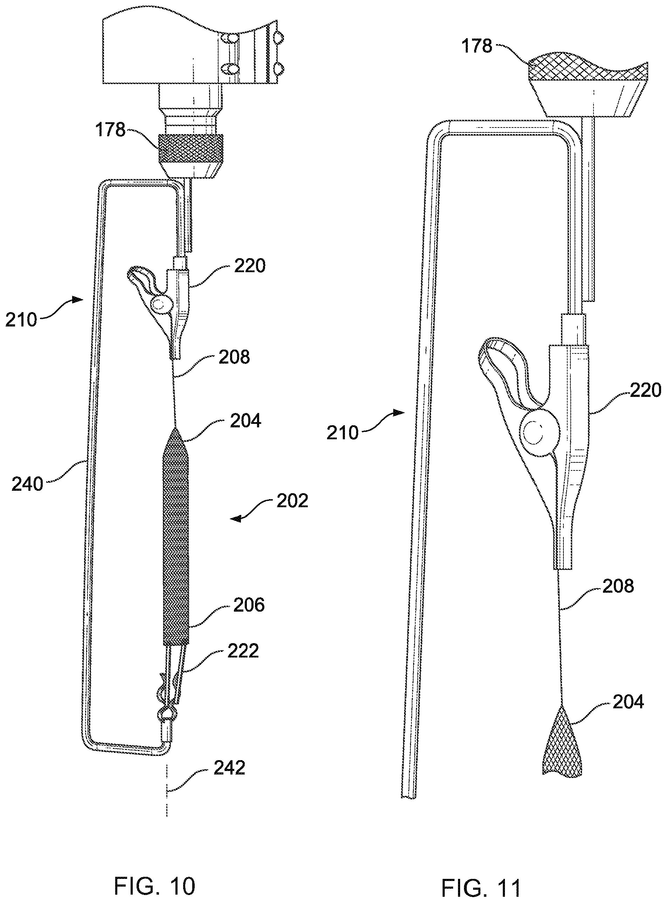

Enlarged views of the mounting apparatus 210, with the stent 202 mounted thereon, are illustrated in Figures 10-12. The stent 202 comprises a tubular braid (which can be similar to any of the herein-described embodiments of the stent 100), with an upper portion 204 and a lower portion 206. As shown in Figures 10-11, in order to facilitate attachment to the mounting apparatus 210, the stent upper portion 204 can be closed and comprise an elongate wire 208 that extends therefrom. For example, in some embodiments, wire 208 can be welded to the stent upper portion 204 to close the stent 202 and form a cone at its upper portion 204. The elongate wire 208 can be engaged by an upper fixture 220 of the mounting apparatus 210. The upper fixture 220 can comprise an alligator clip that can grip the wire 208, or a hook or pin around which the wire 208 can be wound, or other mechanical fastener operative to securely engage the wire 208.

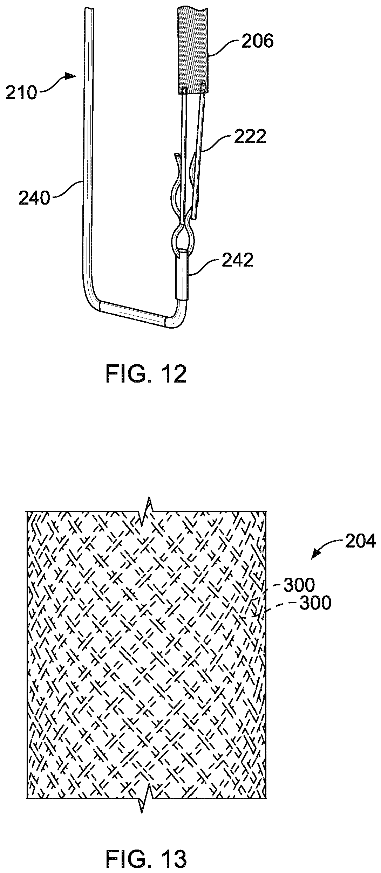

Further, as shown in Figures 10 and 12, the stent lower portion 206 can comprise an open end. The stent lower portion 206 can be engaged by a lower fixture 222 of the mounting apparatus 210. The lower fixture 222 can comprise an alligator clip, a hook, or other mechanical fastener operative to securely engage the stent lower portion 206. For example, Figure 12 illustrates a pair of deflectable prongs that can be inserted into a lumen of the stent 202 and exert a biasing force such that ends of the deflectable prongs engage the inner wall of the stent lower portion 206. Each prong includes near its upper tip an outwardly- and downwardly-projecting barb 223 (Figure 9D) that is received in and projects through a pore of the stent 200, to facilitate a secure grip of the lower portion 206.

The mounting apparatus 210 (see also Figures 9B-9D) can comprise an elongate backbone 240 that interconnects the upper and lower fixtures 220, 222. The upper and lower fixtures 220, 222 can define an axis 242 extending therebetween, along which an axis of the stent 200 can be generally aligned when mounted on the fixtures 220, 222. The elongate backbone 240 can comprise a relatively thin, but nonetheless sufficiently rigid, wire, for example a single stainless steel wire of .055 inch diameter.

The depicted mounting apparatus 210 incorporates a number of design features that enable high-precision coating of the stent 100/202. The upper and lower fixtures 220, 222 are spaced apart so that the stent is held in a longitudinally stretched configuration and the pores are caused to take on the "open" configuration shown in Figure 5C, characterized by the formation of approximately right angles between the filaments. With the pores so opened, any webs can be blown out of the pores without need for a high-pressure air stream which presents a risk of blowing too much of the coating solution off the stent. Substantially no portion of the mounting apparatus 210 protrudes into the lumen of the stent (the sole exception being the tips of the prongs of the lower fixture 222, which extend into a portion of the stent that will be trimmed away after coating is complete). As a result, the mounting apparatus does not interfere with the flow of the air stream 272 through the stent during the knifing process. The only portion of the mounting apparatus 210 that is ever situated between the nozzle 271 and the stent during the knifing process is the backbone 240. However, the effect of this is mitigated by (1) the narrow, thin profile of the backbone 240, (2) the offset between the backbone 240 and the stent, which allows the portion of the stent that is "shadowed" by the backbone to be impacted effectively by the air stream 272 immediately before and after the backbone passes through the air stream, and (3) the fact that the backbone 240 interrupts the air stream 272 only momentarily, once per revolution of the stent, as opposed to the constant airflow interruption that would result from an apparatus member that extends through the stent lumen. The absence of any mounting apparatus member 210 in the stent lumen, and the offset between the backbone 240 and the stent, further contribute to precision coating by reducing or eliminating the possibility of the stent contacting the backbone 240 or any luminally-protruding apparatus member as the stent deflects in reaction to air stream 272 striking the stent. Such contact can wick coating solution away from the stent and degrade the quality of the resulting coating. The tension induced in the stent by virtue of being longitudinally stretched in the mounting apparatus 210 also assists in reducing/eliminating such undesired contact resulting from deflection of the stent. The configuration of the mounting apparatus 210 substantially aligns the axis 242, which is approximately the axis of rotation of the apparatus 210 and the stent when the knifing process is underway, with the central longitudinal axis of the stent. Accordingly, the stent is rotated substantially "on center" during the knifing process, which helps promote an even distance between the stent and the nozzle 271 (and even application of the air stream 272 to the stent) through 360 degrees of rotation of the stent. In addition, the configuration of the lower fixture 222 "places" minimal weight of the mounting apparatus 210 in a location remote from the point of attachment of the apparatus 210 to the chuck 178. It was found that using a clip-and-wire arrangement similar to the upper fixture 220 and upper portion 204 placed too much weight too far from the chuck 178, which induced a "pendulum effect" in the mounting apparatus 210 in reaction to the incident air stream 272, as the apparatus 210 deflected off axis and the resulting back-and-forth motion of the apparatus 210 was magnified by the mass of the relatively heavy lower fixture and its location a relatively long distance away from the chuck 178. A large pendulum effect pulls the stent off-center during knifing and results in uneven application of the air stream 272 to the stent. In comparison, the lighter weight and shorter length of the lower fixture 222 employed in the depicted mounting apparatus 210 reduces the pendulum effect to acceptable levels or eliminates it altogether. The relatively thin profile of the members constituting the mounting apparatus (particularly the backbone 240) also reduces the pendulum effect by reducing the force of the air stream 272 impinging on the mounting apparatus. The depicted lower fixture 222 also keeps the lower portion 206 of the stent open, which allows for more effective draining of excess coating solution from the stent after the stent has been raised out of the coating solution 260, as compared to the conical configuration of the stent upper end 204 employed with the upper fixture 220. The overall cantilevered configuration of the mounting apparatus 210 facilitates dipping the stent in the coating solution 260, and subsequently raising the stent toward the air knife 270 in a simple straight line, while incorporating a brief pre-drying pause before the knifing process begins.

As illustrated in the enlarged view of the dipping procedure shown in Figure 7B, the stent 202 is lowered into the coating solution 260 until the stent upper portion 204 reaches the top surface 262 of the coating material 260, but the stent upper portion 204 is not submersed into the coating material 260. Accordingly, the conical upper portion 204 remains dry. The Applicants discovered that, when the conical upper portion is left dry and uncoated, the coating solution drains more effectively from the stent after it is raised out of the solution 260. This more effective draining of the solution in turn facilitates a neater, more precise blow-off of the solution from the stent and better coating performance.

In a preferred embodiment of the above-described method and apparatus for coating a stent, the dimensions and process parameters shown in Table 1 below can be used for constructing the mounting apparatus 210 and performing the method.

| Table 1 | | | |
|---|---|---|---|
| Mounting Apparatus Dimensions (Figures 9B, 9C, 9D) | | Process Parameters | |
| Dimension | Size | Parameter | Value |
| A | 16.51 cm (6.5") | Air knife pressure | 12 PSI |
| B | 14.22 mm (0.56") | Air knife flow rate | Approx. 26 SLPM |
| C | 17.78 mm (0.7") | Distance from nozzle to stent | 60 mm |
| D | 3.18 cm (1.25") | Nozzle inside diameter | 2.0 mm |
| E | 7.37 cm (2.9") | Rotation speed | 100 RPM |
| F | 6.35 cm (2.5") | Reciprocation speed | 24 mm/s |
| G | 12.7 mm (0.5") | Reciprocation: number of passes through air stream | 5 |
| H | 1.40 mm (0.055") (wire diameter) | Amount of longitudinal stretch of stent, from rest | Approx. 2x |
| I | 8.97 mm (0.353") | Stent braid angle when stretched | Approx. 90 degrees |
| J | 10 degrees | Pre-drying time | 10-15 seconds |
| K | 2.54 cm (1.0") | Drying temperature | 60 degrees C |
| L | 0.81 mm (0.032") (diameter) | Drying time | 15 minutes |
| | | Process ambient temperature and humidity | Room temperature and humidity |
| | | Coating solution temperature | Room temperature |
| | | Pressurized air source | Standard "shop" air with in-line air dryer |

Some of these parameters can be varied in other embodiments. For example, the air knife pressure could be 6-18 PSI, or 10-14 PSI; the distance from the nozzle to the stent could be 20-80 mm, or 40-70 mm; the stent rotation speed could be 50-150RPM, or 80-120 RPM; the reciprocation speed could be 10-40 mm/s, or 18-30 mm/s; the amount of longitudinal stretch of the stent could be 1.5x-3x; the drying temperature could be 50-80 degrees C; and/or the drying time could be 5-60 minutes. Although the air knife 270 is described as blowing air on the stent, other gases could be employed, such as nitrogen, argon or other relatively inert gases.

Other variations of the method and apparatus are possible. For example, multiple nozzles or gas sources could be used in the air knife 270 instead of the single nozzle 271 depicted and described above. The air knife 270 can move during the knifing process while the stent 202 remains stationary, rather than the reverse as described above. The backbone 240 can be made in a constant thickness or diameter that varies somewhat from that specified herein, or the backbone 240 can taper. For example, the backbone 240 can taper from a larger size at its upper end to a smaller size at its lower end.

Further, although dip coating and removal of excess coating material by air knife are discussed above, other coating methods can be implemented. For example, in some embodiments, the stent can be coated using a spray coating process. In spray coating, the coating material may be applied to the device using a spray coater.

Referring now to Figure 13, an enlarged view of the stent 204 is shown in its longitudinally stretched configuration, as achieved when engaged by the mounting apparatus 210 during the coating process. As illustrated, the stent 204 comprises a plurality of filaments 300. Each of the illustrated filaments 300 has been dipped in the coating material and an air stream has been applied to remove excess coating material from the filaments 300. The coating material thus remaining extends only along the filaments 300, without any webbing or delamination occurring.

In this embodiment, the filaments 300 cross each other in a generally orthogonal orientation relative to each other. As illustrated, the filaments 300 cross each other at angles of from about 75° to about 105°, or within from about 0° to about 15° from a right angle.

Generally, according to aspects of embodiments disclosed herein, when the filaments cross each other at substantially orthogonal or right angles (e.g., within about 15° from a right angle), the inter-filament space or crevice area (e.g., the gaps formed between surfaces of overlapping filaments at their crossing point) is minimized, thus reducing the space in which coating material can accumulate in excess of what is required to coat the filament surface. For example, an aspect of embodiments disclosed herein is the realization that the coating may be thicker in inter-filament spaces or crevices due to the surface tension of the coating material. Thus, reducing the inter-filament space can also reduce the amount of coating material captured therein by virtue of the coating material surface tension.

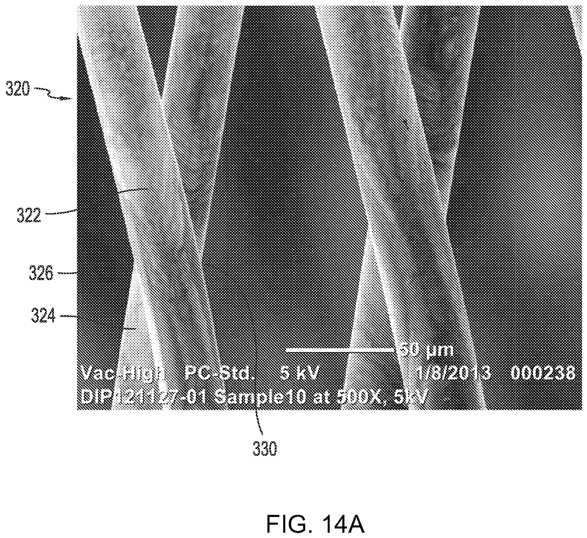

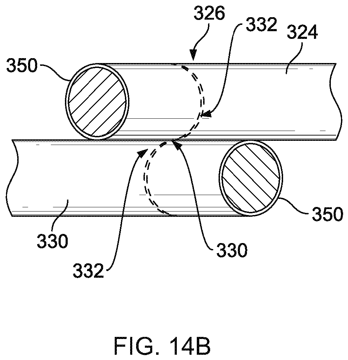

For example, Figures 14A-B illustrate an embodiment of a stent 320 in which filaments 322, 324 overlap at a crossing point 326. Figure 14A shows a coating that demonstrates excellent uniformity, smoothness, and is substantially free of webbing. The filaments 322, 324, which are generally cylindrical, generally have a single contact point 330. Due to the contact and overlap between the filaments 322, 324, an inter-filament or crevice area 332 is formed immediately adjacent to the single contact point 330. During the coating process, excess coating material can accumulate in the crevice areas 332, which can be difficult to remove. However, according to embodiments disclosed herein, the presence and size of inter-filament or crevice areas throughout the braided stent can be minimized by ensuring that the filaments of the portion of the stent to be coated are oriented substantially orthogonally relative to each other. Thus, in some embodiments, a thickness of a coating 350 disposed on the filaments 322, 324 can have a generally constant thickness, even along the crevice areas 332. Further, even with some coating material accumulation, the thickness of the coating 350 disposed in the crevice areas can be less than twice the thickness of the coating 350 disposed on the filaments 322, 324.

Some embodiments of the devices and methods disclosed herein can therefore provide a device, such as a stent or a braided stent, having a coating that is substantially free of webs, and in some embodiments, that also has a flow diverting pore size and/or a flow diverting porosity that is/are exhibited throughout the entire stent, or in a flow diverting portion or section of the stent. In one embodiment, a coated device, stent or section is "substantially free" of webs when any webs that are present are sufficiently few in number so as to not interfere with the function of the device. Alternatively, a coated device, stent or section that is substantially free of webs can be one in which there is webbing present at fewer than 5% of the filament crossing points, and/or at fewer than 5% of the pores. According to the invention webs are present at fewer than 5% of the pores. In another alternative, a coated device, stent or section that is substantially free of webs can be one in which there is webbing present at fewer than 3% of the filament crossing points, and/or at fewer than 3% of the pores. As yet another alternative, a coated device, stent or section can have no webbing at all in any of the filament crossing points, and/or in any of the pores.

Instead of or in addition to the substantial or complete absence of webbing discussed above, the coating of the device, stent or section can be substantially complete. Substantial completeness of coverage can be achieved when the filaments are covered completely along their length within the device, stent or section, with the exception of (a) uncoated areas amounting to less than 5%, or less than 3%, of the outer surface of the filaments, and/or (b) uncoated and/or less-coated (e.g. with fewer than all layers of a multi-layer coating) areas in some or all of the filament crossing points. Alternatively, the coated device, stent or section can be completely coated.

Method for determining the elapsed time before peak thrombin formation: Instead of or in addition to the properties described above relating to lack of webs and/or completeness of coverage, the device, stent or section can be coated with an antithrombogenic coating sufficiently to reduce the thrombogenicity of the coated stent, device, section, etc. as compared to a similar but uncoated stent, device, section, etc. The reduction in thrombogenicity can be significant. Stents coated according to the method disclosed herein have been tested for increased antithrombogenicity via thrombogram, employing the following assay.

| Materials | | |
|---|---|---|
| Description | Information/Purpose | Source |
| Human Platelet Packs | 240 mL stock of citrated aphorized human platelets in plasma (PRP) | Red Cross, Dedham, MA |
| Fluorogenic substrate | For thrombin detection (Z-Gly-Gly-Arg-AMC-HCl, 40mM stock in dimethyl sulfoxide) | Bachem Americas, Inc., Torrance, CA |
| | | Cat#: I-1140 |
| Calcium Chloride (CaCl₂) | For starting coagulation and thrombin generation (1M stock in Distilled Water) | Sigma Aldrich Corp., St. Louis, MO |
| | | Cat#: 223506-2.5KG |
| 4 mm Glass Spheres | Positive control for thrombin generation (assay only) | Fisher Scientific, Waltham, MA |
| | | Cat#: 11-312B |
| Sodium Hypochlorite | Storage of samples post-testing; Cleaning of glass spheres pre-testing | Clorox Co., Oakland, CA |

| | | |
|---|---|---|
| Thrombin Calibrator | For calibration (RFU to Thrombin) in autologous PPP | Diagnostica Stago, Inc., Parsippany, NJ |
| | | Cat#: TS20.00, 1 mL vials |
| HEPES Buffered Saline | For preparation of and serial dilutions of Thrombin stock for calibration | Sigma Aldrich |
| | | Cat#: 51558-50ML |
| Dimethyl Sulfoxide | For substrate stock solution (25 mg/mL) | Sigma Aldrich |
| | | Cat#: D8418-100ML |
| 50 mL Reagent Reservoir Polystyrene | For loading sample solutions into Multipipette Dispenser | Fisher Scientific |
| Centrifuge | For obtaining platelet poor plasma (PPP) from PRP | Eppendorf North America, Hauppauge, NY |
| | | 5810R |
| Rocker | For storing PRP bags until use | Fisher Scientific Labline |
| OptiPlate black/opaque 96-well microplate | For assaying fluorescence of multiple test samples and controls | Fisher Scientific |
| Synergy HT Plate Reader | For simultaneous kinetic fluorescence measurements of multiple samples | Biotek Instruments, Inc., Winooski, VT |
| Particle Counter | For platelet counts (final counts in plasma as required) | Beckman Coulter, Inc., Brea, CA |
| Multipipette Dispenser | For simultaneously dispensing reagents into multiple microplate wells | Mettler Toledo, Inc., Columbus, OH |
| | | Rainin |
| Ruler/Calipers | Dimensions of test samples | MSC |
| Balance | For weighing of stent pieces | Mettler Toledo |

The citrated stock (~240 ml) of platelet rich plasma (PRP) was stored on the rocker (setting: 2) at room temperature for 3 days (up to a maximum of 1 day post printed expiration date). PPP (platelet poor plasma) was prepared on day 1 of obtaining the corresponding PRP stock, as follows: (a) pour 100 mL of PRP stock suspension into two 50 mL centrifuge tubes; (b) centrifuge for 30 minutes at 2900 x *g;* (c) carefully pour out the supernatant-platelet poor plasma (PPP) and filter it using a 0.22 µm syringe-driven filter unit; (d) divide some of the PPP into 5 mL aliquots; (e) store the PPP aliquots at -80°C; and (e) utilize 50 mL of PPP for dilution of autologous PRP for purposes of the assay.

Next, Diluted PRP was prepared as follows: (a) perform platelet count (counting particles within size range of 2µm-4µm for PRP stock solution) in the particle counter by priming the aperture and cleaning the apparatus with purified water, diluting 10 µL of PRP stock in 10 mL of electrolyte solution and initiating the count, and recording the mean platelet count of the PRP stock; (b) dilute PRP to a final count of 200,000 per µL with autologous PPP; and (c) calculate total volume of PRP and PPP needed for assay (per sample = 250 µL).

Samples of stents that were coated according to the method disclosed herein ("test stents") and of identical but bare-metal stents ("bare stents") were prepared as follows: (a) cut sections of each stent to a length of 9 mm; (b) immerse the sections individually in a 1.5 mL centrifuge tube filled with deionized water for 30 minutes; and (c) after wetting, blot out the excess water from each stent section on a kimwipe.

The 96-well microplate was then loaded with the following: (a) test stent sections (5 wells); (b) bare stent sections (5 wells); (c) 4mm glass spheres (5 wells, to function as a positive control); and (d) blanks (5 wells with no samples, serving as a negative control). Only one of a test stent section, bare stent section of glass sphere was placed in any one well. The plate reader was then turned on and allowed to warm up for three minutes, while the associated PC software (Gen 5) was launched. The settings used were: test temperature 25° C; fluorescence reading (excitation (nm): 360/40, emission (nm): 460/40); no shake; kinetic reading every one minute for each well; total experimental time of two hours. Generally, the plate reader was set up for immediate read before preparing the test solutions.

Test solutions were prepared immediately before the analysis as follows. Experimental solution was prepared by adding fluorogenic substrate (25 mg/mL)(40 mM) to Diluted PRP (final count 200,000/µL) in a 15 mL centrifuge tube for a final concentration of 400µM, then adding 1M calcium chloride for a final concentration of 20 mM, and inverting the tube to mix. Calibrator solutions were prepared by (a) adding 1 mL of deionized water to a 1mL vial (as packaged) of the lyophilized thrombin calibrator; (b) preparing 10 mL of HBS-BSA solution (20 mM HEPES, 10 mg/ml bovine serum albumin (BSA), pH 7.4, filtered with the 0.22 µm syringe filter); (c) making four serial dilutions of the calibrator as prepared in step (a), in HBS-BSA (1:2, 1:20, 1:200, 1:2000); (d) adding 100 µL of the fluorogenic substrate to 10 mL of PPP; and (e) mixing five calibrator solutions as follows: (1) by mixing fourteen parts of the substrate-PPP mixture of step (d) with one part of the 1:2 dilution of step (c); (2) by mixing fourteen parts of the substrate-PPP mixture of step (d) with one part of the 1:20 dilution of step (c); (3) by mixing fourteen parts of the substrate-PPP mixture of step (d) with one part of the 1:200 dilution of step (c); (4) by mixing fourteen parts of the substrate-PPP mixture of step (d) with one part of the 1:2000 dilution of step (c); and (5) by mixing fourteen parts of the substrate-PPP mixture of step (d) with one part of the (undiluted) calibrator as prepared in step (a).

The test solutions were transferred to the microplate as follows: (a) 300 µL of the experimental solution in each of the wells containing a test stent section, a bare stent section, or a glass sphere, and in each of the five designated blanks; and (b) 300 µL of calibrator solution in each of ten wells containing no sample (separate from the blank wells), two wells each for each of the five calibrator solutions described above. A multipipette dispenser was used for the experimental solution to ensure that all wells were filled in less than one minute.

The microplate was then placed the reader and the test was run. The coated stents were found to result in a significant delay in peak thrombin formation, as compared to a similar but uncoated stent. In particular, the elapsed time before peak thrombin formation was found to be about 2.5 times that of the similar but uncoated stent. In another experimental run, the elapsed time before peak thrombin formation was found to be about 1.5 times that of the similar but uncoated stent. Accordingly, the time before peak thrombin formation with the coated device, stent, section, etc. can be more than 1.5 times, or more than twice, or about 2.5 times, or about 1.5 times that of a similar but uncoated device, stent, section, etc.

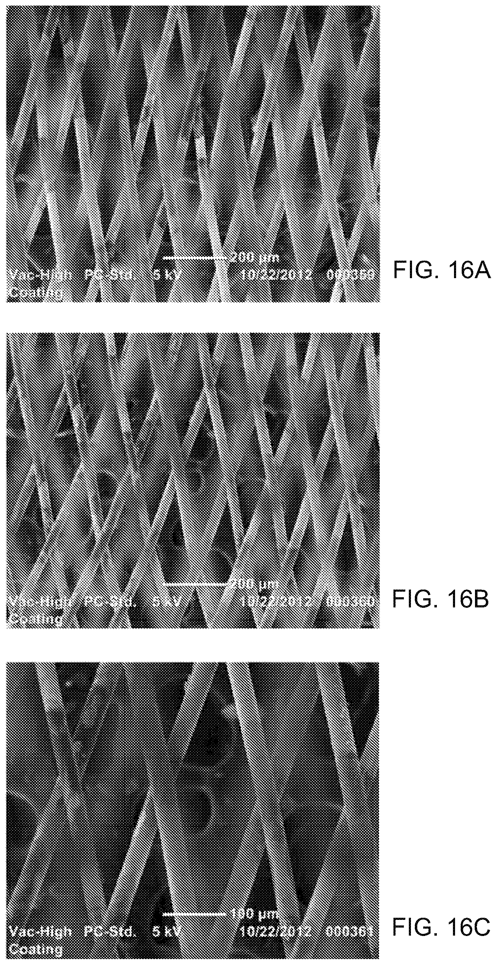

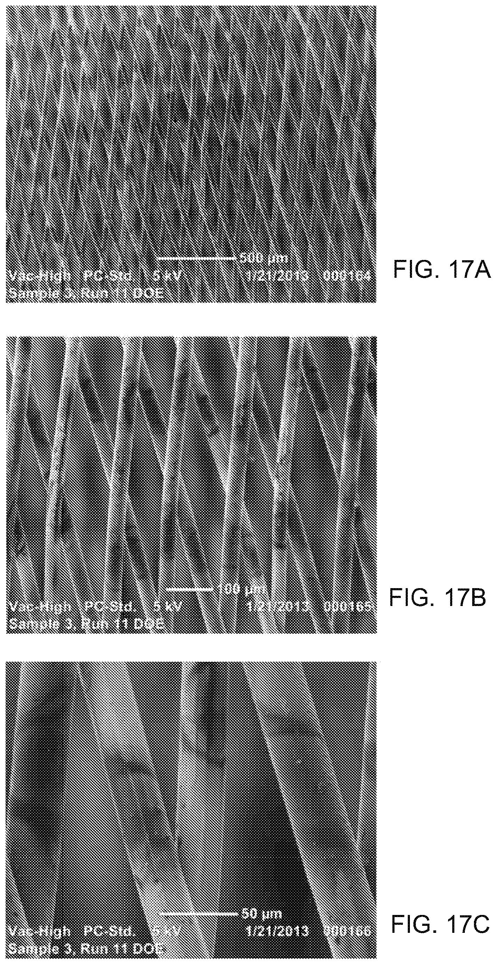

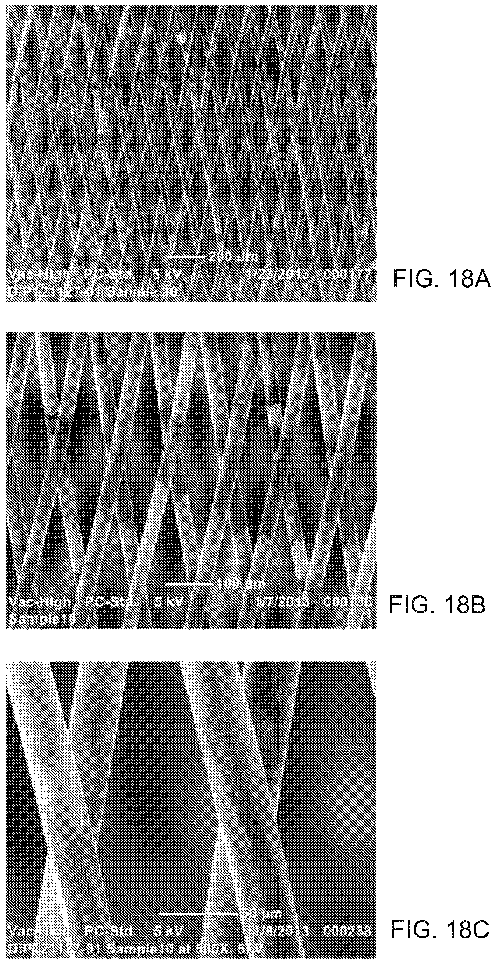

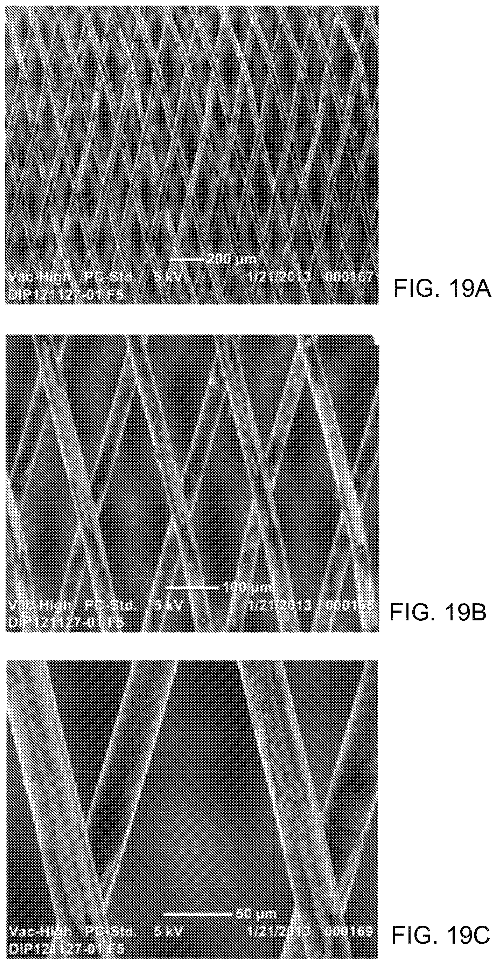

The substantial or complete absence of webbing in the coated device, stent, section, etc. can be observed in SEM (scanning electron microscope) imaging. Figures 16A-19C are SEM images of four different braided stents that were coated using the process disclosed herein and according to the parameters shown in Table 1, each at three levels of magnification: 50X (the "A" images), 150X (the "B" images), and 500X (the "C" images). No webbing can be observed in any of these images. From observation of the images, the pore size of the stent shown in Figures 16C and 19C, as measured by the diameter of an inscribed circle within the pore, is approximately 110 microns. The pore size of the pores illustrated in Figures 17C and 18C is approximately 80 microns.

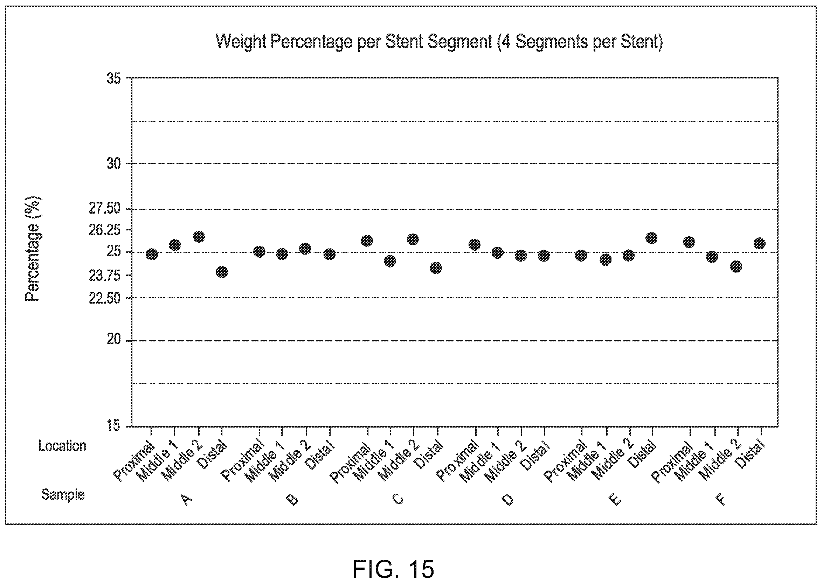

Figures 16A-19C also show complete coverage of the coated stents. Completeness or substantial completeness of coverage, as well as coating uniformity, can also be observed from weight-gain data obtained from coated stents. Figure 15 depicts the results of a weight-gain study performed on six stents that were coated with the process disclosed herein and according to the parameters shown in Table 1. After coating, each stent was cut into four equal-length longitudinal sections (Proximal, Middle #1, Middle #2 and Distal) that were weighed separately to compute a percent weight gain for each stent section. (The bare weight of each section was determined as 1/4 of the pre-coating weight of the stent.) As can be seen in Figure 15, the percent weight gain was highly consistent across all the 24 stent sections. These data indicate completeness or substantial completeness of coverage, as well as uniformity of coverage, because a process that generates piecemeal or gap-laden coverage would be expected to result in high variance in weight gain among the measured sections.

Accordingly, instead of or in addition to the other coating properties discussed herein, the device, stent or section can have a coating weight gain in each of four longitudinal sections of the device, stent or section, wherein the weight gain varies by no more than 2.5 percentage points, or no more than 4 percentage points, or no more than 5 percentage points, between the largest and smallest gains among the four sections.

### Example 1

Braided tubular stents were coated according to the process described herein and the parameters provided in Table 1. Each of the stents was configured as follows: 48 braided filaments, of which 12 were of platinum alloyed with 8% tungsten, with .0012 inch filament diameter, 12 were of 35NLT, with .0012 inch filament diameter, and 24 were of 35NLT, with .0014 inch filament diameter; overall outside diameter 5.2 mm and longitudinal picks per inch of 275, both dimensions prevailing when in an expanded, unconstrained and unstretched condition.

The stents were provided in their "bare metal" condition, and prepared as follows. First, they were washed in 99.5% acetone for five minutes in an ultrasonic cleaner, and then washed in 99% isopropyl alcohol (IPA) in an ultrasonic cleaner, in two separate five-minute IPA wash stages. After washing, the stents were rinsed in distilled water and then dried in an oven at 60 degrees C for 30 minutes.

A coating solution of 2-Methacryloyloxyethyl phosphorylcholine (MPC) was prepared by dissolving 2.0 grams of MPC (LIPIDURE^{™}-CM2056) in 200 milliliters of ethanol, and provided in a beaker at room temperature. The coating process was then performed on each of the stents, according to the description provided herein and the parameters shown in Table 1. After completion of the process and trimming, the stents could be described as tubular braided stents, open at each end with a lumen extending from one end to the other, and with a coating of MPC over the entirety of the stent filaments.

Figures 16A-19C are SEM images of four stents coated according to this Example 1. As described previously, the images indicate that the stents are coated completely and with no observable webbing in the pores.

Figure 15 depicts the results of a weight gain study performed on six stents coated according to this Example 1. Again, the weight-gain data shows that the stents were coated completely or substantially completely, and with high uniformity.

Stents coated according to this Example 1 were tested for increased antithrombogenicity via thrombogram, employing the assay described above. The coated stents were found to result in a significant delay in peak thrombin formation, as compared to an identical but uncoated stent. In particular, the elapsed time before peak thrombin formation was found to be about 2.5 times the time observed with the identical but uncoated stent.

### Methods of Treatment

As mentioned elsewhere herein, the present disclosure also includes methods of treating a vascular condition, such as an aneurysm or intracranial aneurysm, with any of the embodiments of the coated stents disclosed herein. The coated, low-thrombogenicity stent could be deployed across the neck of an aneurysm and its flow-diverting properties employed to reduce blood flow between the aneurysm and the parent vessel, cause the blood inside the aneurysm to thrombose and lead to healing of the aneurysm.

Significantly, the low-thrombogenicity stents disclosed herein can facilitate treatment of a large population of patients for whom flow-diverter therapy has not been previously possible. Such patients are those who have previously suffered from a hemorrhagic aneurysm or who have been diagnosed as being at risk for hemorrhage from an aneurysm or other vascular anatomy such as from the intracranial arterial system. These patients cannot currently be treated with commercially available flow-diverting stents because those stents are bare metal, braided stents whose implantation requires the patient to take blood-thinning medication (typically aspirin and PLAVIX^{™} (clopidogrel)) for a long period of time following implantation. The purpose of the blood-thinning medication is to counteract the tendency of the bare-metal stent to cause thrombus (blood clots) to form in the patient's vasculature. However, for a patient who has suffered or is at risk of intracranial hemorrhage, taking the blood-thinning medication can cause, or put the patient at higher risk of, such a hemorrhage. Low-thrombogenicity flow-diverting stents, such as the coated stents disclosed herein, can make flow-diverter therapy possible for patients who cannot tolerate blood-thinning medication because the reduced thrombogenicity can reduce or eliminate the need for blood thinners.

In order to implant any of the coated stents disclosed herein, the stent can be mounted in a delivery system. Suitable delivery systems are disclosed in U.S. Patent Application No. 13/692,021, filed December 3, 2012, titled METHODS AND APPARATUS FOR LUMINAL STENTING; and in U.S. Patent No. 8,273,101, issued September 25, 2012, titled SYSTEM AND METHOD FOR DELIVERING AND DEPLOYING AN OCCLUDING DEVICE WITHIN A VESSEL. In particular, these documents' teachings regarding braided stent delivery systems and methods may be employed to deliver any of the coated stents disclosed herein in the same manner, to the same bodily location(s), and using the same components as are disclosed in both incorporated documents.

Generally, the delivery system can include an elongate core assembly that supports or contains the stent, and both components can be slidably received in a lumen of a microcatheter or other elongate sheath for delivery to any region to which the distal opening of the microcatheter can be advanced. The core assembly is employed to advance the stent through the microcatheter and out the distal end of the microcatheter so that the stent is allowed to self-expand into place in the blood vessel, across an aneurysm or other treatment location. The core assembly can comprise a wire (e.g. a guidewire), a tube (e.g. a hypotube such as a spiral-cut or slotted hypotube), or a combination of a wire and a tube. The delivery system may employ a polymeric member that engages an inner wall of the tubular body and enables longitudinal force transmission from the core assembly to the tubular body. Such a polymeric member can be mounted on the wire and/or tube.

A treatment procedure can begin with obtaining percutaneous access to the patient's arterial system, typically via a major blood vessel in a leg or arm. A guidewire can be placed through the percutaneous access point and advanced to the treatment location, which can be in an intracranial artery. The microcatheter is then advanced over the guidewire to the treatment location and situated so that a distal open end of the guidewire is adjacent to the treatment location. The guidewire can then be withdrawn from the microcatheter and the core assembly, together with the stent mounted thereon or supported thereby, can be advanced through the microcatheter and out the distal end thereof. The stent can then self-expand into apposition with the inner wall of the blood vessel. Where an aneurysm is being treated, the stent is placed across the neck of the aneurysm so that a sidewall of the stent (e.g. a section of the braided tube) separates the interior of the aneurysm from the lumen of the parent artery. Once the stent has been placed, the core assembly and microcatheter are removed from the patient. The stent sidewall can now perform a flow-diverting function on the aneurysm, thrombosing the blood in the aneurysm and leading to healing of the aneurysm.

Because of the low-thrombogenic properties of the coated stents disclosed herein, certain additional aspects of the methods of treatment are possible. For example, the patient can be one who has previously suffered from, or who has been diagnosed as being at risk, of hemorrhage from an aneurysm or other arterial anatomy such as the intracranial arterial system. The patient can be prescribed a reduced regimen of blood-thinning medication as compared to the regimen that would be necessary for patient who received an otherwise similar but uncoated flow-diverting stent. The regimen can be "reduced" in the sense that the patient takes a lower dosage, fewer medications, less powerful medications, follows a lower dosage frequency, and/or takes medication for a shorter period of time following implantation of the stent, or otherwise. Alternatively, the patient may be prescribed no blood thinning medication at all.

The devices and methods discussed herein are not limited to the coating of stents, but may include any number of other implantable devices. Treatment sites may include blood vessels and areas or regions of the body such as organ bodies.

Although the detailed description contains many specifics, these should not be construed as limiting the scope of the subject technology but merely as illustrating different examples and aspects of the subject technology. It should be appreciated that the scope of the subject technology includes other embodiments not discussed in detail above. Various other modifications, changes and variations may be made in the arrangement, operation and details of the method and apparatus of the subject technology disclosed herein without departing from the scope of the present disclosure, as defined in the appended claims. Unless otherwise expressed, reference to an element in the singular is not intended to mean "one and only one" unless explicitly stated, but rather is meant to mean "one or more." In addition, it is not necessary for a device to address every problem that is solvable by different embodiments of the disclosure in order to be encompassed within the scope of the disclosure.

## Claims

1. A medical device (100) for treating an aneurysm, comprising:
a tubular member comprising a plurality of braided filaments that form a sidewall and a plurality of pores in the sidewall that are sized to inhibit flow of blood through the sidewall into an aneurysm to a degree sufficient to lead to thrombosis and healing of the aneurysm when the tubular member is positioned in a blood vessel and adjacent to the aneurysm; and
an anti-thrombogenic material comprising an anti-thrombogenic polymer distributed over the tubular member such that the pores are substantially free of webs formed by the material, such that webs are present at fewer than 5% of the pores;
wherein the pores have an average pore size that is less than or equal to about 500 microns, the tubular member exhibits an elapsed time before peak thrombin formation that is at least 1.5 times the elapsed time of an identical, uncoated tubular member wherein the elapsed time is determined according to the method described in the section named "Method for determining the elapsed time before peak thrombin formation", and wherein the tubular member is expandable to an expanded diameter of 2 millimetres (mm) or more.

2. The medical device of Claim 1, wherein the pores have an average pore size that is less than or equal to about 320 microns.

3. The medical device of Claim 2, wherein the pores have an average pore size that is measured using an inscribed circle diameter.

4. The medical device of Claim 1, wherein the distribution of the anti-thrombogenic material is substantially complete over the tubular member.

5. The medical device of Claim 4, wherein the anti-thrombogenic material is generally uniform over the tubular member.

6. The medical device of any Claim 1, wherein the distribution of the anti-thrombogenic material is substantially complete over at least a circumferential section of the tubular member that is 5 mm or more in length.

7. The medical device of Claim 1, wherein the anti-thrombogenic polymer comprises MPC.

8. The medical device of Claim 1, wherein the tubular member comprises a heat-set metallic braid.

9. The medical device of Claim 1, wherein the tubular member is self-expanding.

10. The medical device of Claim 1, wherein the tubular member has an open proximal end, an open distal end, and forms a lumen extending from the proximal end to the distal end.

11. The medical device of Claim 1, wherein the tubular member exhibits an elapsed time before peak thrombin formation that is at least 2.5 times the elapsed time of an identical, uncoated tubular member.

12. The medical device of Claim 1, wherein the tubular member has an unexpanded diameter smaller than the expanded diameter, and the unexpanded diameter is 5mm or less.

13. The medical device of Claim 12, wherein the unexpanded diameter is 3mm or less.

14. A delivery system for treating an aneurysm, the system comprising:
an elongate core assembly comprising a tube, a wire, or a combination of a tube and a wire; and
the medical device of Claim 1 engaged with the core assembly so as to be movable via the core assembly.

## Patentansprüche

1. Medizinische Vorrichtung (100) zum Behandeln eines Aneurysmas, umfassend:
ein rohrförmiges Element, das eine Vielzahl von geflochtenen Filamenten, die eine Seitenwand bilden, und eine Vielzahl von Poren in der Seitenwand umfasst, die so bemessen sind, dass sie den Blutfluss durch die Seitenwand in ein Aneurysma in einem Ausmaß hemmen, das ausreicht, um zu einer Thrombose und Heilung des Aneurysmas zu führen, wenn das rohrförmige Element in einem Blutgefäß und angrenzend an das Aneurysma positioniert ist; und
ein antithrombogenes Material, das ein antithrombogenes Polymer umfasst, das über das rohrförmige Element derart verteilt ist, dass die Poren im Wesentlichen frei von durch das Material gebildeten Stegen sind, sodass Stege mit weniger als 5 % der Poren vorliegen;
wobei die Poren eine durchschnittliche Porengröße aufweisen, die kleiner oder gleich etwa 500 Mikrometer ist, das rohrförmige Element eine verstrichene Zeit vor der Spitzenthrombinbildung aufweist, die mindestens das 1,5-fache der verstrichenen Zeit eines identischen, unbeschichteten rohrförmigen Elements beträgt, wobei die verstrichene Zeit gemäß dem in dem Abschnitt "Verfahren zum Bestimmen der verstrichenen Zeit vor der Spitzenthrombinbildung" beschriebenen Verfahren bestimmt wird, und wobei das rohrförmige Element auf einen expandierten Durchmesser von 2 Millimetern (mm) oder mehr expandierbar ist.

2. Medizinische Vorrichtung nach Anspruch 1, wobei die Poren eine durchschnittliche Porengröße von weniger als oder gleich etwa 320 Mikrometer aufweisen.

3. Medizinische Vorrichtung nach Anspruch 2, wobei die Poren eine durchschnittliche Porengröße aufweisen, die unter Verwendung eines eingeschriebenen Kreisdurchmessers gemessen wird.

4. Medizinische Vorrichtung nach Anspruch 1, wobei die Verteilung des antithrombogenen Materials im Wesentlichen vollständig über das rohrförmige Element erfolgt.

5. Medizinische Vorrichtung nach Anspruch 4, wobei das antithrombogene Material im Allgemeinen gleichmäßig über das rohrförmige Element verteilt ist.

6. Medizinische Vorrichtung nach einem der Ansprüche 1, wobei die Verteilung des antithrombogenen Materials im Wesentlichen vollständig über mindestens einen Umfangsabschnitt des rohrförmigen Elements erfolgt, der 5 mm oder mehr lang ist.

7. Medizinische Vorrichtung nach Anspruch 1, wobei das antithrombogene Polymer MPC umfasst.

8. Medizinische Vorrichtung nach Anspruch 1, wobei das rohrförmige Element ein hitzegehärtetes Metallgeflecht umfasst.

9. Medizinische Vorrichtung nach Anspruch 1, wobei das rohrförmige Element von selbst expandiert.

10. Medizinische Vorrichtung nach Anspruch 1, wobei das rohrförmige Element ein offenes proximales Ende, ein offenes distales Ende aufweist und ein Lumen bildet, das sich von dem proximalen Ende zu dem distalen Ende erstreckt.

11. Medizinische Vorrichtung nach Anspruch 1, wobei das rohrförmige Element eine verstrichene Zeit vor der Spitzenthrombinbildung aufweist, die mindestens das 2,5-fache der verstrichenen Zeit eines identischen unbeschichteten rohrförmigen Elements beträgt.

12. Medizinische Vorrichtung nach Anspruch 1, wobei das rohrförmige Element einen nicht expandierten Durchmesser aufweist, der kleiner als der expandierte Durchmesser ist, und der nicht expandierte Durchmesser 5 mm oder weniger beträgt.

13. Medizinische Vorrichtung nach Anspruch 12, wobei der nicht expandierte Durchmesser 3 mm oder weniger beträgt.

14. Abgabesystem zum Behandeln eines Aneurysmas, das System umfassend:
eine längliche Kernanordnung, die ein Rohr, einen Draht oder eine Kombination aus einem Rohr und einem Draht umfasst; und
wobei die medizinische Vorrichtung nach Anspruch 1 mit der Kernanordnung in Eingriff steht, um über die Kernanordnung bewegbar zu sein.

## Revendications

1. Dispositif médical (100) permettant de traiter un anévrisme, comprenant :
un élément tubulaire comprenant une pluralité de filaments tressés qui forment une paroi latérale et une pluralité de pores dans la paroi latérale qui sont dimensionnés pour inhiber un écoulement de sang à travers la paroi latérale dans un anévrisme à un degré suffisant pour conduire à une thrombose et une guérison de l'anévrisme lorsque l'élément tubulaire est positionné dans un vaisseau sanguin et adjacent à l'anévrisme ; et
un matériau anti-thrombogène comprenant un polymère anti-thrombogène distribué sur l'élément tubulaire de telle sorte que les pores sont sensiblement dépourvus de bandes formées par le matériau, de telle sorte que des bandes sont présentes à hauteur de moins de 5 % des pores ;
dans lequel les pores ont une taille moyenne de pore qui est inférieure ou égale à environ 500 microns, l'élément tubulaire présente un temps écoulé avant la formation de thrombine maximale qui est au moins 1,5 fois le temps écoulé d'un élément tubulaire non revêtu identique dans lequel le temps écoulé est déterminé selon la méthode décrite dans la section dénommée « Méthode permettant de déterminer le temps écoulé avant la formation de thrombine maximale », et dans lequel l'élément tubulaire est expansible à un diamètre expansé de 2 millimètres (mm) ou plus.

2. Dispositif médical selon la revendication 1, dans lequel les pores ont une taille moyenne de pore qui est inférieure ou égale à environ 320 micromètres.

3. Dispositif médical selon la revendication 2, dans lequel les pores ont une taille moyenne de pore qui est mesurée à l'aide d'un diamètre de cercle inscrit.

4. Dispositif médical selon la revendication 1, dans lequel la distribution du matériau anti-thrombogène est sensiblement complète sur l'élément tubulaire.

5. Dispositif médical selon la revendication 4, dans lequel le matériau anti-thrombogène est généralement uniforme sur l'élément tubulaire.

6. Dispositif médical selon l'une quelconque de la revendication 1, dans lequel la distribution du matériau anti-thrombogène est sensiblement complète sur au moins une section circonférentielle de l'élément tubulaire d'une longueur de 5 mm ou plus.

7. Dispositif médical selon la revendication 1, dans lequel le polymère anti-thrombogène comprend de la MPC.

8. Dispositif médical selon la revendication 1, dans lequel l'élément tubulaire comprend une tresse métallique thermodurcie.

9. Dispositif médical selon la revendication 1, dans lequel l'élément tubulaire est auto-expansible.

10. Dispositif médical selon la revendication 1, dans lequel l'élément tubulaire a une extrémité proximale ouverte, une extrémité distale ouverte, et forme une lumière s'étendant de l'extrémité proximale à l'extrémité distale.

11. Dispositif médical selon la revendication 1, dans lequel l'élément tubulaire présente un temps écoulé avant la formation de thrombine maximale qui est au moins 2,5 fois le temps écoulé d'un élément tubulaire non revêtu identique.

12. Dispositif médical selon la revendication 1, dans lequel l'élément tubulaire a un diamètre non expansé inférieur au diamètre expansé, et le diamètre non expansé est de 5 mm ou moins.

13. Dispositif médical selon la revendication 12, dans lequel le diamètre non expansé est de 3 mm ou moins.

14. Système d'administration permettant de traiter un anévrisme, le système comprenant :
un ensemble central allongé comprenant un tube, un fil, ou une combinaison d'un tube et d'un fil ; et
le dispositif médical selon la revendication 1 en prise avec l'ensemble central de manière à être mobile par l'intermédiaire de l'ensemble central.
